# EUROPÄISCHE PATENTSCHRIFT

(11) **EP 3 625 280 B1**
(45) Veröffentlichungstag und Bekanntmachung des Hinweises auf die Patenterteilung: **07.07.2021**
(21) Anmeldenummer: 18720640.4
(22) Anmeldetag: 07.05.2018
(51) Int. Cl.: C07C 265/14, C08G 18/75, C08G 18/80, C08G 18/32, C07C 263/16

(54) **POLYISOCYANAT (P) UND VERFAHREN ZU DESSEN HERSTELLUNG**
POLYISOCYANATE (P) AND METHOD FOR THE PRODUCTION THEREOF
POLYISOCYANATE (P) ET SON PROCÉDÉ DE FABRICATION

(30) Priorität: 17.05.2017 EP 17171588
(43) Veröffentlichungstag der Anmeldung: 25.03.2020
(73) Patentinhaber: BASF SE, 67056 Ludwigshafen (DE)
(72) Erfinder: LUCAS, Frederic, 67056 Ludwigshafen (DE); PANCHENKO, Alexander, 67056 Ludwigshafen (DE); BREUER, Klaus, 67056 Ludwigshafen (DE); HETTCHE, Frank, 67056 Ludwigshafen (DE); THAM, Chee Kean, Hong Kong (CN)
(86) Internationale Anmeldenummer: PCT/EP2018/061644
(87) Internationale Veröffentlichungsnummer: WO 2018/210592

(56) Entgegenhaltungen:
- WO-A1-2009/053307
- DE-A1- 19 547 205
- GB-A- 1 409 536
- US-A1- 2015 183 922

## Beschreibung

Die vorliegende Erfindung betrifft ein Polyisocyanat (P) der allgemeinen Formel (I), das Verfahren zur Herstellung des Polyisocyanats (P) der allgemeinen Formel (I) sowie die Verwendung des Polyisocyanats (P) der allgemeinen Formel (I) als Vernetzungsreagenz in Klarlacken. Das Verfahren zur Herstellung des Polyisocyanats (P) der allgemeinen Formel (I) umfasst die Umsetzung eines Reaktionsgemisches (RG), welches mindestens ein zyklisches Isocyanat der allgemeinen Formeln (IIIa), (IIIb), (IIIc), (IIId), (IIIe) und/oder (IIIf) sowie mindestens einen Alkohol, der mindestens zwei Hydroxygruppen aufweist, enthält, unter Erhalt des Polyisocyanats (P) der allgemeinen Formel (I).

Organische Polyisocyanate spielen im Bereich der Kunststoffe, Pressmassen sowie Verklebungen und insbesondere auf dem Lacksektor eine entscheidende Rolle. Aufgrund der Toxizität von organischen Isocyanaten werden diese in der Regel mit Polyalkoholen umgesetzt, um Produkte mit einem geringeren Dampfdruck zu erhalten. Dabei entstehen im Wesentlichen niedermolekulare und lagerstabile Polyurethane, die weiterhin über reaktive Isocyanatgruppen verfügen und die physiologisch deutlich weniger bedenklich sind als die organischen Isocyanat-Ausgangsmaterialien. Die so erhaltenen Polyurethane weisen allerdings im Vergleich zum organischen Isocyanat-Ausgangsmaterial oft einen geringeren Gehalt an Isocyanatgruppen auf, weshalb mehr Material eingesetzt werden muss, damit die gewünschte Stoffmenge an Isocyanatgruppen für weitere Umsetzungen vorliegt.

Die Herstellung von Polyisocyanaten aus organischen Isocyanaten und Alkoholen ist im Stand der Technik beschrieben.

So offenbart US-A 2015/0183922 eine Polyisocyanatzusammensetzung, die auf bis(Isocyanatomethyl)cyclohexan und Trimethylolpropan basiert. Die Polyisocyanatzusammensetzung enthält dabei sowohl das Reaktionsprodukt aus einem Molekül Trimethylolpropan mit drei Molekülen bis(Isocyanatomethyl)cyclohexan als auch das Reaktionsprodukt aus zwei Molekülen Trimethylolpropan mit fünf Molekülen bis(Isocyanatomethyl)cyclohexan. Diese Reaktionsprodukte werden als Quervernetzer neben weiteren aliphatischen, aromatischen und araliphatischen Polyisocyanaten und Polyisocyanatderivaten eingesetzt.

WO 2009/071533 offenbart allophanatgruppenhaltige Polyisocyanate, die durch Umsetzung von Isophorondiisocyanat mit einem Polyalkohol oder einem hydroxygruppenhaltigen Amin in Gegenwart eines Katalysators erhalten werden. Die allophanatgruppenhaltigen Polyisocyanate werden in zweikomponentigen Polyurethanlacken neben mindestens einer Komponente eingesetzt, die gegenüber Isocyanat reaktive Gruppen enthält. Die allophanatgruppenhaltigen Polyisocyanate dienen dabei als Vernetzerkomponente.

US-A 3,595,838 offenbart polyurethanhaltige Lackzusammensetzungen, die ein Polyesterpolyol und eine Isocyanatverbindung enthalten. Die Isocyanatverbindung ist ein Addukt aus Trimethylolpropan und bis(Isocyanatomethyl)benzol. Zur Herstellung dieses Addukts wird das Isocyanat in einem sieben- bis sechzehnfachen Überschuss gegenüber Trimethylolpropan eingesetzt, damit das Addukt ausreichend löslich in Lösungsmitteln und kompatibel mit dem Polyesterpolyol ist.

In DE-A 1090196 wird ein Verfahren zur Herstellung von Mono- oder Polyisocyanaten mit geringem Dampfdruck offenbart, in dem ein oder mehrere Alkohole mit einem großen Überschuss an Diisocyanaten umgesetzt werden. Als Alkohole eignen sich gesättigte und ungesättigte Glykole und Polyole.

DE-A 953012 offenbart ein Verfahren zur Herstellung von Polyisocyanaten, bei dem ähnlich wie in DE-A 1090196 mehrwertige, niedermolekulare Alkohole mit einem Überschuss an aliphatischen und/oder aromatischen Diisocyanaten umgesetzt werden. Als Alkohole werden drei- oder vierwertige Alkohole wie Trimethylolpropan, Glyzerin, Pentaerythrit oder Triethanolamin eingesetzt, die gegebenenfalls im Gemisch mit zweiwertigen Alkoholen wie Ethylenglykol oder Butylenglykol verwendet werden.

Analog dazu beschreibt auch DE-B 870400 die Herstellung von organischen Polyisocyanaten durch Umsetzung von drei- und/oder vierwertigen Alkoholen, die im Gemisch mit zweiwertigen Alkoholen vorliegen können, mit mindestens einem Mol Diisocyanat. Als Diisocyanate werden aliphatische Diisocyanate wie Tetramethylendiisocyanat oder Hexamethylendiisocyanat angeführt. Die in DE-B 870400 angeführten aromatischen Diisocyanate umfassen beispielsweise Toluylendiisocyanat und Diphenylendiisocyanat.

Aufgabe der vorliegenden Erfindung ist somit die Bereitstellung eines neuen Polyisocyanats sowie eines Verfahrens zu dessen Herstellung.

Diese Aufgabe wird gelöst durch das Polyisocyanat (P) der allgemeinen Formel (I): 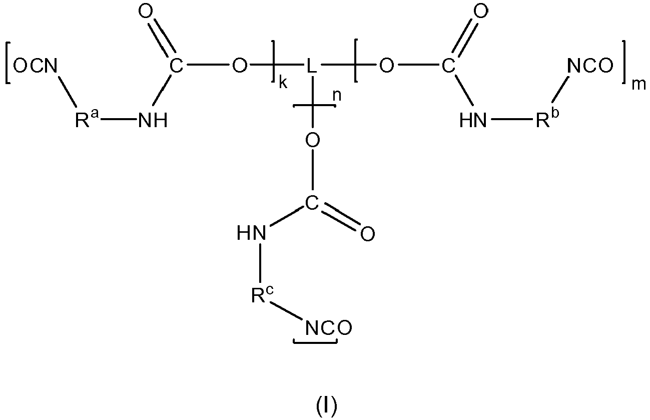 in der
- k, m, n: unabhängig voneinander 0, 1, 2 oder 3 sind, wobei die Summe von k, m und n mindestens 2 und höchstens 6 ergibt; und
- L: ein linearer oder verzweigter organischer Rest mit höchstens 14 Kohlenstoffatomen ist, der gegebenenfalls mindestens ein Stickstoffatom und/oder mindestens ein Sauerstoffatom enthalten kann,
- R^{a}, R^{b}, R^{c}: unabhängig voneinander ausgewählt sind aus Resten der allgemeinen Formeln (IIa), (IIb), (IIc), (IId), (IIe) und/oder (IIf): 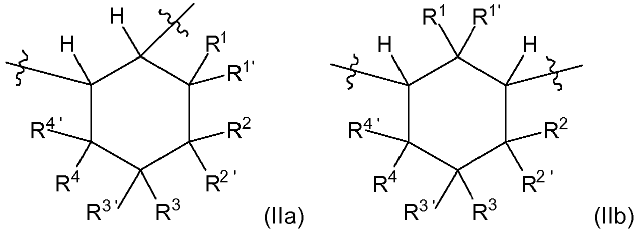 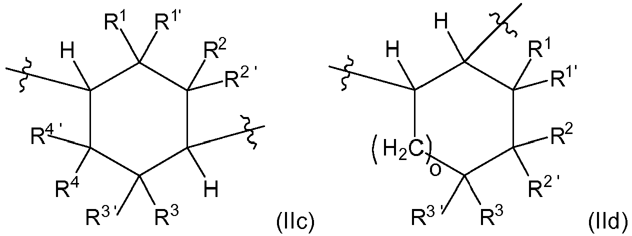 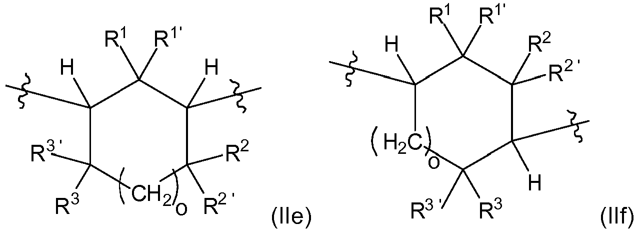
in denen
- o: 0 bis 10 ist, und
- R¹, R^{1'}, R², R^{2'}, R³, R^{3'}, R⁴, R^{4'}: unabhängig voneinander ausgewählt sind aus der Gruppe bestehend aus H, OR⁵ und unsubstituiertem oder zumindest monosubstituiertem C₁-C₁₀-Alkyl, C₅-C₁₂-Cycloalkyl, C₂-C₁₀-Alkenyl und C₆-C₁₄-Aryl, wobei R⁵ C₁-C₁₀-Alkyl ist.

Es wurde überraschend gefunden, dass Polyisocyanate (P) der allgemeinen Formel (I) im Vergleich zu den im Stand der Technik beschriebenen Polyisocyanaten einen höheren Gehalt an Isocyanatgruppen bei vergleichbarer Viskosität aufweisen. Dadurch wird im Vergleich zu dem im Stand der Technik beschriebenen Verfahren eine geringere Menge an Polyisocyanat für weitere Umsetzungen benötigt, was die Herstellungskosten von Folgeprodukten reduziert.

Im Rahmen der vorliegenden Erfindung wird unter einem Polyisocyanat eine organische Verbindung verstanden, welche zwei oder mehr Isocyanatgruppen (-NCO) enthält.

Im Rahmen der vorliegenden Erfindung wird unter einem Polyurethan eine organische Verbindung verstanden, welche zwei oder mehr Urethangruppen (-O-CO-NH-) enthält.

Im Rahmen der vorliegenden Erfindung bedeuten Definitionen wie "C₁-C₁₀-Alkyl", wie beispielsweise für die Reste R¹ bis R^{4'} in den Formeln (IIa) bis (IIf) definiert, dass dieser Substituent ein Alkylrest mit 1 bis 10 Kohlenstoffatomen sein kann. Dieser kann, falls nicht anders angegeben, linear oder verzweigt sein, aber auch gleichzeitig, anteilig, alle beiden Formen aufweisen. Beispiele für entsprechende Alkylreste sind Methyl, Ethyl, Propyl, Isopropyl, Butyl, tert-Butyl, sec-Butyl, Pentyl, Hexyl, Heptyl, Octyl, Nonyl und Decyl.

Im Rahmen der vorliegenden Erfindung bedeuten Definitionen wie "C₅-C₁₂-Cycloalkyl", wie beispielsweise für die Reste R¹ bis R^{4'} in den Formeln (IIa) bis (IIf) definiert, dass dieser Substituent ein Cycloalkylrest mit 5 bis 12 Kohlenstoffatomen sein kann. Beispiele für entsprechende Cycloalkylreste sind Cyclopentyl, Cyclohexyl, Cycloheptyl, Cyclooctyl, Norbornyl oder Adamantyl.

Im Rahmen der vorliegenden Erfindung bedeuten Definitionen wie "C₆-C₁₄-Aryl", wie beispielsweise für die die Reste R¹ bis R^{4'} in den Formeln (IIa) bis (IIf) definiert, dass dieser Substituent ein Aromat mit 6 bis 14 Kohlenstoffatomen sein kann. Bei dem Aromaten kann es sich um einen monozyklischen, bizyklischen oder polyzyklischen Aromaten handeln. Im Fall von polyzyklischen Aromaten können einzelne Ringe ganz oder teilweise gesättigt sein. Beispiele für entsprechende Arylreste sind Phenyl, Naphthyl, Anthracyl oder Phenanthryl.

Im Rahmen der vorliegenden Erfindung bedeuten Definitionen wie "C₂-C₁₀-Alkenyl", wie für die Reste R¹ bis R^{4'} in den Formeln (IIa) bis (IIf) definiert, dass dieser Substituent ein Alkenylrest mit 2 bis 10 Kohlenstoffatomen sein kann. Dieser Kohlenstoffrest ist vorzugsweise einfach ungesättigt, gegebenenfalls kann er aber auch zwei- oder mehrfach ungesättigt sein. Vorzugsweise ist C₂-C₁₀-Alkenyl im Rahmen der vorliegenden Erfindung Vinyl, 1-Allyl, 2-Allyl, 3-Allyl, cis- oder trans-2-Butenyl oder ω-Butenyl.

Die Bezeichnung "unsubstituiert" bedeutet im Rahmen der vorliegenden Erfindung, dass C₁-C₁₀-Alkyl, C₅-C₁₂-Cycloalkyl, C₂-C₁₀-Alkenyl oder C₆-C₁₄-Aryl keine weiteren Substituenten außer Wasserstoff (H) aufweisen.

Die Bezeichnung "zumindest monosubstituiert" bedeutet im Rahmen der vorliegenden Erfindung, dass C₁-C₁₀-Alkyl, C₅-C₁₂-Cycloalkyl oder C₆-C₁₄-Aryl genau einen Substituenten oder auch zwei oder mehrere Substituenten aufweisen kann. Die Substituenten können gleich oder verschieden sein und sind C₁-C₁₀-Alkyl, die wie vorstehend definiert sind.

Die vorliegende Erfindung wird nachfolgend im Detail erläutert.

Das Polyisocyanat (P) weist die allgemeine Formel (I) auf: 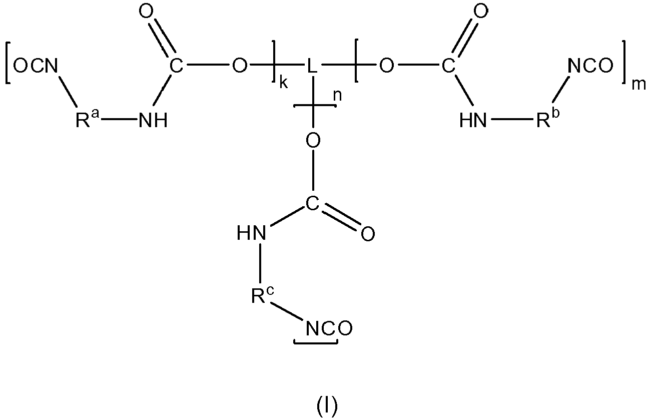 in der
- k, m, n: unabhängig voneinander 0, 1, 2 oder 3 sind, wobei die Summe von k, m und n mindestens 2 und höchstens 6 ergibt; und
- L: ein linearer oder verzweigter organischer Rest mit höchstens 14 Kohlenstoffatomen ist, der gegebenenfalls mindestens ein Stickstoffatom und/oder mindestens ein Sauerstoffatom enthalten kann,
- R^{a}, R^{b}, R^{c}: unabhängig voneinander ausgewählt sind aus Resten der allgemeinen Formeln (IIa), (IIb), (IIc), (IId), (IIe) und/oder (IIf): 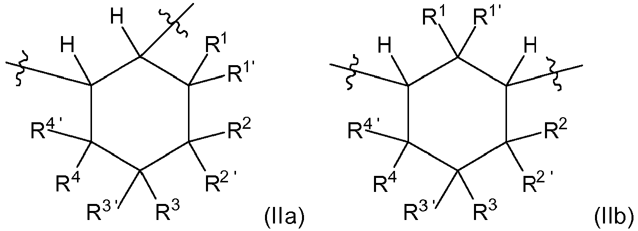 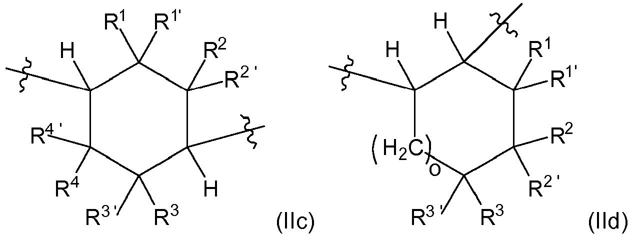 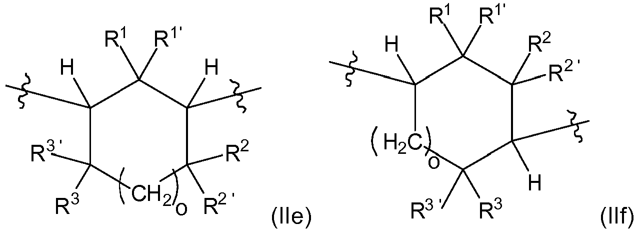
in denen
- o: 0 bis 10 ist, und
- R¹, R¹', R², R^{2'}, R³, R^{3'}, R⁴, R^{4'}: unabhängig voneinander ausgewählt sind aus der Gruppe bestehend aus H, OR⁵ und unsubstituiertem oder zumindest monosubstituiertem C₁-C₁₀-Alkyl, C₅-C₁₂-Cycloalkyl, C₂-C₁₀-Alkenyl und C₆-C₁₄-Aryl, wobei R⁵ C₁-C₁₀-Alkyl ist.

k, m und n sind unabhängig voneinander 0, 1, 2 oder 3. Die Summe von k, m und n ergibt dabei mindestens 2 und höchstens 6, besonders bevorzugt höchstens 4.

Bevorzugt ist k mindestens 2, besonders bevorzugt mindestens 3 und höchstens 4, und m und n sind 0. Die Werte für k, m und n können im statistischen Mittel ungeradzahlige Werte annehmen, sie sind dann aber bezogen auf jedes einzelne Molekül des Isocyanats (P) der allgemeinen Formel (I) geradzahlig.

L ist erfindungsgemäß ein linearer oder verzweigter organischer Rest mit höchstens 14 Kohlenstoffatomen, der gegebenenfalls mindestens ein Stickstoffatom und/oder mindestens ein Sauerstoffatom enthalten kann, L enthält mindestens ein, vorzugsweise mindestens zwei Kohlenstoffatome.

Die Anzahl der Stickstoff- und/oder Sauerstoffatome ist nicht beschränkt. In der Regel beträgt sie nicht mehr als 5 in dem Rest, bevorzugt nicht mehr als 4 und mehr bevorzugt nicht mehr als 3, besonders bevorzugt enthält L maximal ein Stickstoff- oder ein Sauerstoffatom. Weiterhin befindet sich zwischen zwei Stickstoff- und/oder Sauerstoffatomen in der Regel mindestens ein Kohlenstoffatom, bevorzugt mindestens zwei Kohlenstoffatome. In einer bevorzugten Ausführungsform enthält L keine Heteroatome (also N oder O).

L ist vorzugsweise ein linearer oder verzweigter, (k + m + n)-wertiger, bevorzugt zweibis sechswertiger, besonders bevorzugt zwei- bis vierwertiger und ganz besonders bevorzugt dreiwertiger organischer Rest, der entsprechend seiner Wertigkeit mit zwei bis sechs, besonders bevorzugt mit zwei bis vier und ganz besonders bevorzugt mit drei Urethangruppen verbunden ist. Unter der "Wertigkeit" wird im Rahmen der vorliegenden Anmeldung die Anzahl an Urethangruppen-haltigen Resten verstanden, die an einen organischen Rest L substituiert sind.

Bevorzugt ist L ein linearer oder verzweigter aliphatischer, cycloaliphatischer oder aromatischer Rest mit höchstens 14 Kohlenstoffatomen, der gegebenenfalls mindestens ein Stickstoffatom und/oder mindestens ein Sauerstoffatom enthalten kann. Geeignete lineare oder verzweigte aliphatische, cycloaliphatische oder aromatische Reste leiten sich von entsprechenden (k + m + n)-wertigen Alkoholen ab, die weiter unten ausführlicher beschrieben werden.

Mehr bevorzugt ist L ein linearer oder verzweigter aliphatischer oder cycloaliphatischer Rest mit höchstens 14 Kohlenstoffatomen, der gegebenenfalls mindestens ein Stickstoffatom und/oder mindestens ein Sauerstoffatom enthalten kann.

Noch mehr bevorzugt ist L ein linearer oder verzweigter aliphatischer Rest mit höchstens 10 Kohlenstoffatomen, der gegebenenfalls mindestens ein Stickstoffatom und/oder mindestens ein Sauerstoffatom enthalten kann.

Besonders bevorzugt ist L ein linearer oder verzweigter aliphatischer Rest mit höchstens 6 Kohlenstoffatomen, der gegebenenfalls mindestens ein Sauerstoffatom enthalten kann.

Bei den Resten R^{a}, R^{b} und R^{c} der allgemeinen Formeln (IIa), (IIb), (IIc), (IId), (IIe) und/oder (IIf) handelt es sich um Molekülfragmente innerhalb des Polyisocyanats (P). Dem Fachmann ist dabei klar, dass die geschlängelten Linien in den allgemeinen Formeln (IIa), (IIb), (IIc), (IId), (IIe) und (IIf) das Ende des entsprechenden Molekülfragments zeigen und dass das entsprechende Molekülfragment an diesen Stellen mit den Stickstoffatomen der Isocyanatgruppen beziehungsweise den Stickstoffatomen der Urethangruppen des Polyisocyanats (P) verknüpft ist.

Vorzugsweise sind R^{a}, R^{b} und R^{c} in der allgemeinen Formel (I) unabhängig voneinander ausgewählt aus Resten der allgemeinen Formeln (IIa), (IIb), (IIc), (IId), (IIe) und/oder (IIf), in denen o 0, 2 oder 3 ist und R¹, R¹', R², R^{2'}, R³, R^{3'}, R⁴ und R^{4'} unabhängig voneinander ausgewählt sind aus der Gruppe bestehend aus H und unsubstituiertem oder zumindest monosubstituiertem C₁-C₁₀-Alkyl.

Mehr bevorzugt sind R^{a}, R^{b} und R^{c} in der allgemeinen Formel (I) unabhängig voneinander ausgewählt aus Resten der allgemeinen Formeln (IIa), (IIb) und/oder (IIc), in denen R¹, R¹', R², R^{2'}, R³, R^{3'}, R⁴ und R^{4'} unabhängig voneinander ausgewählt sind aus der Gruppe bestehend aus H, Methyl, Ethyl und Propyl.

Noch mehr bevorzugt sind R^{a}, R^{b} und R^{c} in der allgemeinen Formel (I) ausgewählt aus Resten der allgemeinen Formeln (IIa), (IIb) und/oder (IIc), in denen R¹, R^{1'}, R², R^{2'}, R³, R^{3'}, R⁴ und R^{4'} unabhängig voneinander ausgewählt sind aus der Gruppe bestehend aus H, Methyl und Ethyl, wobei mindestens ein Rest R¹, R^{1'}, R², R^{2'}, R³, R^{3'}, R⁴ oder R^{4'} Methyl oder Ethyl, vorzugsweise Methyl, sind.

Besonders bevorzugt sind R^{a}, R^{b} und R^{c} in der allgemeinen Formel (I) Reste der allgemeinen Formel (IIb), in der R¹, R¹', R², R^{2'}, R³, R^{3'}, R⁴ und R^{4'} unabhängig voneinander ausgewählt sind aus der Gruppe bestehend aus H, Methyl und Ethyl, wobei mindestens ein Rest und höchstens 3 Reste R¹, R^{1'}, R², R^{2'}, R³, R^{3'}, R⁴ oder R^{4'}Methyl oder Ethyl, vorzugsweise Methyl, sind.

Ganz besonders bevorzugt sind R^{a}, R^{b} und R^{c} in der allgemeinen Formel (I) ein Rest der allgemeinen Formel (IIb), in der R¹, R^{1'}, R² und R^{2'} unabhängig voneinander ausgewählt sind aus der Gruppe bestehend aus H und Methyl, wobei mindestens ein Rest und höchstens 3 Reste R¹, R^{1'}, R² und R^{2'} Methyl sind, und in der R³, R^{3'}, R⁴ und R^{4'} H sind.

In einer bevorzugten Ausführungsform weist das Polyisocyanat (P) die allgemeine Formel (I) auf, in der
- k, m, n: unabhängig voneinander 0, 1, 2 oder 3 sind, wobei die Summe von k, m und n mindestens 2 und höchstens 6 ergibt; und
- L: ein linearer oder verzweigter aliphatischer, cycloaliphatischer oder aromatischer Rest mit höchstens 14 Kohlenstoffatomen ist, der gegebenenfalls mindestens ein Stickstoffatom und/oder mindestens ein Sauerstoffatom enthalten kann,
- R^{a}, R^{b}, R^{c}: unabhängig voneinander ausgewählt sind aus Resten der allgemeinen Formeln (IIa), (IIb), (IIc), (IId), (IIe) und/oder (IIf), in denen
- o: 0, 2 oder 3 ist, und
- R¹, R^{1'}, R², R^{2'}, R³, R^{3'}, R⁴, R^{4'}: unabhängig voneinander ausgewählt sind aus der Gruppe bestehend aus H und C₁-C₁₀-Alkyl.

In einer mehr bevorzugten Ausführungsform weist das Polyisocyanat (P) die allgemeine Formel (I) auf, in der
- k, m, n: unabhängig voneinander 0, 1, 2 oder 3 sind, wobei die Summe von k, m und n mindestens 2 und höchstens 6 ergibt; und
- L: ein linearer oder verzweigter aliphatischer oder cycloaliphatischer Rest mit höchstens 14 Kohlenstoffatomen ist, der gegebenenfalls mindestens ein Stickstoffatom und/oder mindestens ein Sauerstoffatom enthalten kann,
- R^{a}, R^{b}, R^{c}: unabhängig voneinander ausgewählt sind aus Resten der allgemeinen Formeln (IIa), (IIb) und/oder (IIc), in denen
- R¹, R^{1'}, R², R^{2'}, R³, R^{3'}, R⁴, R^{4'}: unabhängig voneinander ausgewählt sind aus der Gruppe bestehend aus H, Methyl, Ethyl und Propyl.

In einer noch mehr bevorzugten Ausführungsform weist das Polyisocyanat (P) die allgemeine Formel (I) auf, in der
- k: 2 oder 3 ist, und
- m, n: 0 sind, und
- L: ein linearer oder verzweigter aliphatischer Rest mit höchstens 10 Kohlenstoffatomen ist, der gegebenenfalls mindestens ein Stickstoffatom und/oder mindestens ein Sauerstoffatom enthalten kann,
- R^{a}: ausgewählt ist aus Resten der allgemeinen Formeln (IIa), (IIb) und/oder (IIc), in denen
- R¹, R^{1'}, R², R^{2'}, R³, R^{3'}, R⁴, R^{4'}: unabhängig voneinander ausgewählt sind aus der Gruppe bestehend aus H, Methyl und Ethyl,
wobei mindestens ein Rest R¹, R^{1'}, R², R^{2'}, R³, R^{3'}, R⁴ oder R^{4'} Methyl oder Ethyl, vorzugsweise Methyl, ist.

In einer besonders bevorzugten Ausführungsform weist das Polyisocyanat (P) die allgemeine Formel (I) auf, in der
- k: 3 ist, und
- m, n: 0 sind, und
- L: ein linearer oder verzweigter aliphatischer Rest mit höchstens 6 Kohlenstoffatomen ist, der gegebenenfalls mindestens ein Sauerstoffatom enthalten kann,
- R^{a}: ein Rest der allgemeinen Formel (IIb) ist, in der
- R¹, R^{1'}, R², R^{2'}, R³, R^{3'}, R⁴, R^{4'}: unabhängig voneinander ausgewählt sind aus der Gruppe bestehend aus H, Methyl und Ethyl,
wobei mindestens ein Rest und höchstens 3 Reste R¹, R^{1'}, R², R^{2'}, R³, R^{3'}, R⁴ oder R^{4'} Methyl oder Ethyl, vorzugsweise Methyl, sind.

In einer ganz besonders bevorzugten Ausführungsform weist das Polyisocyanat (P) die allgemeine Formel (I) auf, in der
- k: 3 ist und
- m, n: 0 sind, und
- L: ein linearer oder verzweigter aliphatischer Rest mit höchstens 6 Kohlenstoffatomen ist, der gegebenenfalls mindestens ein Sauerstoffatom enthalten kann,
- R^{a}: ein Rest der allgemeinen Formel (IIb) ist, in der
- R¹, R^{1'}, R², R^{2'}, R³, R^{3'}, R⁴, R^{4'}: unabhängig voneinander ausgewählt sind aus der Gruppe bestehend aus H, Methyl und Ethyl,
wobei mindestens ein Rest und höchstens 3 Reste R¹, R^{1'}, R², R^{2'}, R³, R^{3'}, R⁴ oder R^{4'} Methyl oder Ethyl, vorzugsweise Methyl, sind.

In einer am meisten bevorzugten Ausführungsform weist das Polyisocyanat (P) die allgemeine Formel (IV) auf: 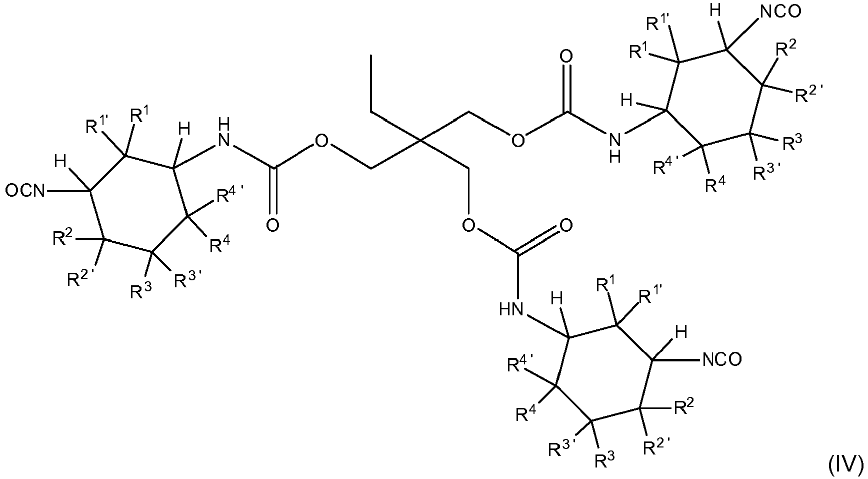 in der
- R¹, R^{1'}, R², R^{2'}: unabhängig voneinander ausgewählt sind aus der Gruppe bestehend aus H und Methyl, wobei mindestens ein Rest und höchstens zwei Reste R¹, R^{1'}, R² oder R^{2'} Methyl sind, und
- R³, R^{3'}, R⁴, R^{4'}: H sind.

Ein weiterer Gegenstand der vorliegenden Erfindung ist außerdem ein Verfahren zur Herstellung des Polyisocyanats (P), umfassend die Umsetzung eines Reaktionsgemisches (RG), enthaltend die folgenden Komponenten (a) und (b):
(a) mindestens ein zyklisches Isocyanat der allgemeinen Formeln (lila), (IIIb), (IIIc), (IIId), (IIIe) und/oder (IIIf): 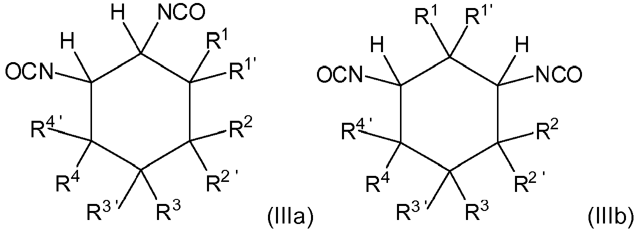 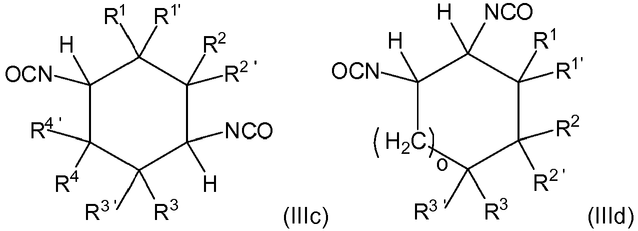 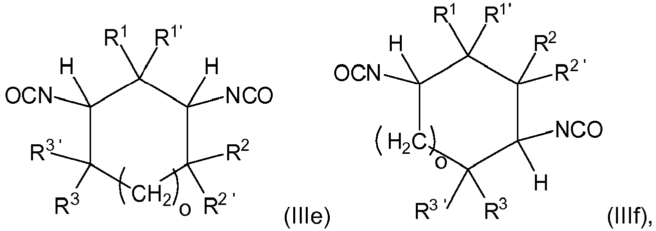 in denen
   - o: 0 bis 10 ist, und
   - R¹, R^{1'}, R², R^{2'}, R³, R^{3'}, R⁴, R^{4'}: unabhängig voneinander ausgewählt sind aus der Gruppe bestehend aus H, OR⁵ und unsubstituiertem oder zumindest monosubstituiertem C₁-C₁₀-Alkyl, C₅-C₁₂-Cycloalkyl, C₂-C₁₀-Alkenyl und C₆-C₁₄-Aryl, wobei R⁵ C₁-C₁₀-Alkyl ist, und
(b) mindestens ein Alkohol, der mindestens zwei Hydroxygruppen aufweist,
unter Erhalt des Polyisocyanats (P).

Die Herstellung des Polyisocyanats (P) erfolgt durch das nachfolgend im Detail erläuterte Verfahren.

Im erfindungsgemäßen Verfahren wird ein Reaktionsgemisch (RG), welches mindestens ein zyklisches Isocyanat der allgemeinen Formeln (lila), (IIIb), (IIIc), (IIId), (IIIe) und/oder (IIIf) (Komponente (a)) sowie mindestens einen Alkohol, der mindestens zwei Hydroxygruppen aufweist (Komponente (b)), enthält, umgesetzt unter Erhalt einer Zusammensetzung (Z), welche das Polyisocyanat (P) der allgemeinen Formel (I) enthält.

Die Komponenten (a) und (b) werden nachfolgend im Detail erläutert.

Das Reaktionsgemisch (RG) enthält als Komponente (a) mindestens ein zyklisches Isocyanat der allgemeinen Formeln (IIIa), (IIIb), (IIIc), (IIId), (IIIe) und/oder (IIIf).

Die Bezeichnung "Komponente (a)" und "mindestens ein zyklisches Isocyanat der allgemeinen Formeln (IIIa), (IIIb), (IIIc), (IIId), (IIIe) und/oder (IIIf)" werden im Rahmen der vorliegenden Erfindung synonym verwendet.

Die Bezeichnung "mindestens ein zyklisches Isocyanat" bezieht sich dabei sowohl auf genau ein zyklisches Isocyanat als auch auf Mischungen von zwei oder mehreren zyklischen Isocyanaten der allgemeinen Formeln (IIIa), (IIIb), (IIIc), (IIId), (IIIe) und/oder (IIIf).

Geeignete zyklische Isocyanate sowie Verfahren zu deren Herstellung sind dem Fachmann prinzipiell bekannt und können beispielsweise durch Phosgenierung der ihnen zugrunde liegenden Amine hergestellt werden.

Das im erfindungsgemäßen Verfahren eingesetzte mindestens eine zyklische Isocyanat weist mindestens eine der allgemeinen Formeln (IIIa), (IIIb), (IIIc), (IIId), (IIIe) und/oder (IIIf) auf: 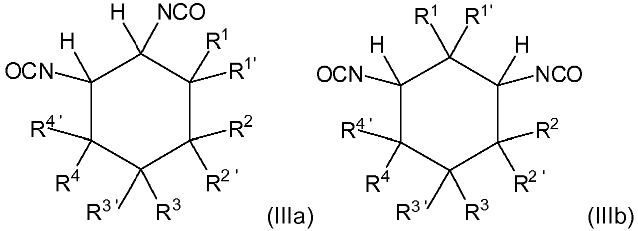 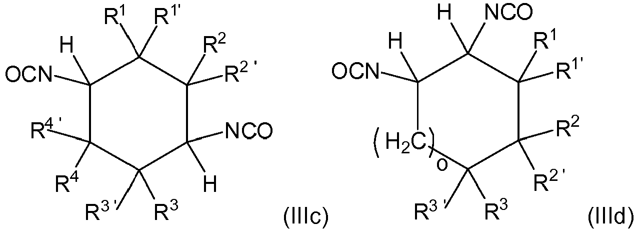 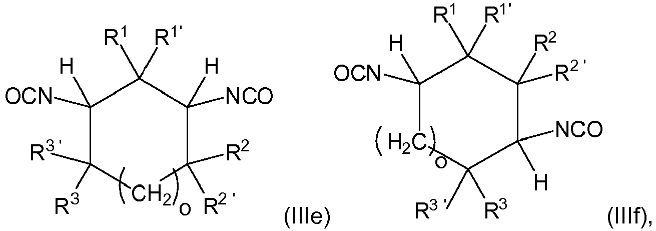 in denen
- o: 0 bis 10 ist, und
- R¹, R^{1'}, R², R^{2'}, R³, R^{3'}, R⁴, R^{4'}: unabhängig voneinander ausgewählt sind aus der Gruppe bestehend aus H, OR⁵ und unsubstituiertem oder zumindest monosubstituiertem C₁-C₁₀-Alkyl, C₅-C₁₂-Cycloalkyl, C₂-C₁₀-Alkenyl und C₆-C₁₄-Aryl, wobei R⁵ C₁-C₁₀-Alkyl ist.

Die Reste R¹ bis R^{4'} in dem mindestens einen zyklischen Isocyanat der allgemeinen Formeln (IIIa), (IIIb), (IIIc), (IIId), (IIIe) und/oder (IIIf) sind prinzipiell identisch mit den Resten R¹ bis R^{4'} in den Formeln (IIa) bis (IIf) des Polyisocyanats (P) der allgemeinen Formeln (I) oder (IV). Folglich gelten für die Reste R¹ bis R^{4'} in den allgemeinen Formeln (IIIa) bis (IIIf) die vorstehenden Ausführungen und Bevorzugungen für die Reste R¹ bis R^{4'} in den Formeln (IIa) bis (IIf) des Polyisocyanats (P) der allgemeinen Formel (I) oder (IV) entsprechend.

Dem Fachmann ist folglich klar, dass für den Erhalt eines bestimmten Polyisocyanats (P) als Komponente (a) mindestens ein zyklisches Isocyanat der allgemeinen Formeln (IIIa), (IIIb), (IIIc), (IIId), (IIIe) und/oder (IIIf) eingesetzt werden muss, welches sich in den Resten R^{1'} bis R^{4'} nicht von den entsprechenden Resten R^{1'} bis R^{4'} in den Formeln (IIa) bis (IIf) im Polyisocyanat (P) der allgemeinen Formeln (I) oder (IV) unterscheidet.

Vorzugsweise ist die Komponente (a) ausgewählt aus 1,3-Diisocyanatocyclohexan, 1,3-Diisocyanato-2-methylcyclohexan, 1,3-Diisocyanato-4-methylcyclohexan, 1,3-Diisocyanato-5-methylcyclohexan, 1,3-Diisocyanato-2-isopropylcyclohexan, 1,3-Diisocyanato-4-isopropylcylohexan, 1,3-Diisocyanato-5-isopropylcyclohexan, 1,3-Diisocyanato2,4-dimethylcyclohexan, 1,3-Diisocyanato-2,4-diethylcyclohexan, 1,3-Diisocyanato-2,4diethyl-6-methylcyclohexan, 1,3-Diisocyanato-2-methyl-4,5-diethylcyclohexan, 1,3-Diisocyanato-2,4,6-triisopropylcyclohexan oder 1,3-Diisocyanato-2,4,6-tributylcyclohexan.

Mehr bevorzugt ist die Komponente (a) ausgewählt aus 1,3-Diisocyanatocyclohexan, 1,3-Diisocyanato-2-methylcyclohexan, 1,3-Diisocyanato-4-methylcyclohexan, 1,3-Diisocyanato-5-methylcyclohexan, 1,3-Diisocyanato-2,4-dimethylcyclohexan, 1,3-Diisocyanato-2,4-diethylcyclohexan und 1,3-Diisocyanato-2,4-diethyl-6-methylcyclohexan,

Noch mehr bevorzugt enthält die Komponente (a) mindestens 80 Gew.-%, bevorzugt mindestens 90 Gew.-% und besonders bevorzugt mindestens 98 Gew.-% mindestens eines zyklischen Isocyanats ausgewählt aus 1,3-Diisocyanato-2-methylcyclohexan oder 1,3-Diisocyanato-4-methylcyclohexan, bezogen auf das Gesamtgewicht der Komponente (a) im Reaktionsgemisch (RG).

Besonders bevorzugt besteht die Komponente (a) aus mindestens einem zyklischen Isocyanat ausgewählt aus 1,3-Diisocyanato-2-methylcyclohexan oder 1,3-Diisocyanato-4-methylcyclohexan.

Vorzugsweise ist die Komponente (a) eine Mischung aus 1,3-Diisocyanato-2-methylcyclohexan und 1,3-Diisocyanato-4-methylcyclohexan. Die Gewichtsanteile von 1,3-Diisocyanato-2-methylcyclohexan und 1,3-Diisocyanato-4-methylcyclohexan in dieser Mischung können dabei prinzipiell beliebig sein.

Bevorzugt enthält die Komponente (a) in diesem Fall 50 bis 95 Gew.-% an 1,3-Diisocyanato-4-methylcyclohexan und 5 bis 50 Gew.-% 1,3-Diisocyanato-2-methylcyclohexan, bezogen auf das Gesamtgewicht der Komponente (a).

Die im erfindungsgemäßen Verfahren eingesetzte Komponente (a) weist bevorzugt ein Molekulargewicht im Bereich 130 bis 500 g/mol, besonders bevorzugt von 140 bis 350 g/mol auf.

Das Reaktionsgemisch (RG) enthält als Komponente (b) mindestens einen Alkohol, der mindestens zwei Hydroxygruppen aufweist. Unter der Bezeichnung "Hydroxygruppe" wird im Rahmen der vorliegenden Erfindung -OH verstanden.

Die Bezeichnung "Komponente (b)" und "mindestens ein Alkohol, der mindestens zwei Hydroxygruppen aufweist" werden dabei im Rahmen der vorliegenden Erfindung synonym verwendet.

Die Bezeichnung "mindestens ein Alkohol" bezieht sich dabei auf genau einen Alkohol als auch auf Mischungen von zwei oder mehreren verschiedenen Alkoholen, die mindestens zwei Hydroxygruppen aufweisen. Unter der Bezeichnung "mindestens zwei Hydroxygruppen" wird im Rahmen der vorliegenden Erfindung verstanden, dass die Komponente (b) genau zwei Hydroxygruppen als auch drei oder mehr Hydroxygruppen aufweisen kann. Geeignete Alkohole, die mindestens zwei Hydroxygruppen aufweisen, sind dem Fachmann im Allgemeinen bekannt.

Die Komponente (b) ist vorzugweise ausgewählt aus Ethylenglykol, 1,1-Dimethylethylenglykol, 1,2-Propandiol, 1,3-Propandiol, 2-Methyl-1,3-Propandiol, 2-Ethyl-1,3-Propandiol, 2-Butyl-2-ethyl-1,3-Propandiol, 1,4-Butandiol, 2-Ethyl-1,4-butandiol, 1,5-Pentandiol, 2-Methyl-1,5-pentandiol, Neopentylglykol, 1,6-Hexandiol, 2-Ethyl-1,3-Hexandiol, 2,4-Diethyloktan-1,3-diol, Diethylenglykol, Triethylenglykol, Tetraethylenglykol, Pentaethylenglykol, Dipropylenglykol, Tripropylenglykol, Trimethylolethan, Trimethylolpropan, Trimethylolbutan, Ditrimethylolpropan, Pentaerythrit, Dipentaerythrit, Glyzerin, Cyclohexan-1,2-diol, Cyclohexan-1,3-diol, Cyclohexan-1,4-diol, 1,1-Bis(hydroxymethyl)cyclohexan, 1,2-Bis(hydroxymethyl)cyclohexan, 1,3-Bis(hydroxymethyl)cyclohexan, 1,4-Bis(hydroxymethyl)cyclohexan, 2,2-Bis(4-hydroxycyclohexyl)propan, Erythrit, Threit, Xylit, Adonit (Ribit), Arabit (Lyxit), Sorbit, Mannit, Dulcit (Galactit), Maltit, Isomalt, Diglyzerin, Dimethylolpropan, Dipentaerythritol, Ribose, Arabinose, Glucose, Mannose, Galactose, Fructose, Brenzcatechin, Catechol, Hydrochinon, Pyrogallol, Hydroxyhydrochinon, Phloroglucin, Hydroxypivalinsäureneopentylglykolester, Triethanolamin, Tripropanolamin, 1,3,5-tris(2-Hydroxyethyl)-cyanursäure, Polytetrahydrofuran mit einem Molekulargewicht zwischen 162 und 4500 g/mol, bevorzugt 250 bis 2000 g/mol, Poly-1,3-propandiol oder Poly-1,2-propandiol mit einem Molekulargewicht zwischen 134 und 2000 g/mol oder Polyethylenglykol mit einem Molekulargewicht zwischen 238 und 2000 g/mol.

Die Komponente (b) ist mehr bevorzugt ausgewählt aus Ethylenglykol, 1,2-Propandiol, 1,3-Propandiol, 1,4-Butandiol, 1,5-Pentandiol, 1,6-Hexandiol, Diethylenglykol, Triethylenglykol, Trimethylolpropan, Trimethylolbutan, Pentaerythrit, Glyzerin, Erythrit, Threit, Xylit, Ribit, Arabit, Sorbit, Mannit, Galactit, Diglyzerin, Dimethylolpropan oder Dipentaerythritol.

Noch mehr bevorzugt ist die Komponente (b) ausgewählt aus Ethylenglykol, 1,2-Propandiol, 1,3-Propandiol, 1,4-Butandiol, Diethylenglykol, Trimethylolethan, Trimethylolpropan, Trimethylolbutan und Glyzerin.

Besonders bevorzugt ist die Komponente (b) ausgewählt aus Ethylenglykol, 1,2-Propandiol, 1,3-Propandiol, 1,4-Butandiol, Diethylenglykol, Trimethylolpropan und Glyzerin.

Vorzugsweise enthält die Komponente (b) mindestens 80 Gew.-%, bevorzugt mindestens 90 Gew.-% und besonders bevorzugt mindestens 98 Gew.-% mindestens eines Alkohols ausgewählt aus der Gruppe bestehend aus Ethylenglykol, 1,2-Propandiol, 1,3-Propandiol, 1,4-Butandiol, Diethylenglykol, Trimethylolethan, Trimethylolpropan, Trimethylolbutan und Glyzerin, bezogen auf das Gesamtgewicht der Komponente (b) im Reaktionsgemisch (RG), wobei Ethylenglykol, 1,2-Propandiol, 1,3-Propandiol, 1,4-Butandiol, Diethylenglykol, Trimethylolpropan und Glyzerin besonders bevorzugt sind.

Mehr bevorzugt besteht die Komponente (b) aus mindestens einem Alkohol ausgewählt aus der Gruppe bestehend aus Ethylenglykol, 1,2-Propandiol, 1,3-Propandiol, 1,4-Butandiol, Diethylenglykol, Trimethylolethan, Trimethylolpropan, Trimethylolbutan und Glyzerin, wobei Ethylenglykol, 1,2-Propandiol, 1,3-Propandiol, 1,4-Butandiol, Diethylenglykol, Trimethylolpropan und Glyzerin besonders bevorzugt sind.

In einer bevorzugten Ausführungsform umfasst die Komponente (b) mindestens zwei Alkohole, wobei mindestens ein Alkohol zwei Hydroxygruppen aufweist und mindestens ein Alkohol mindestens drei Hydroxygruppen aufweist. Vorzugsweise weist der Alkohol, welcher mindestens drei Hydroxygruppen aufweist, höchstens sechs Hydroxygruppen auf, besonders bevorzugt höchstens vier Hydroxygruppen und ganz besonders bevorzugt genau drei Hydroxygruppen.

In dieser Ausführungsform ist der Alkohol, der zwei Hydroxygruppen aufweist, vorzugsweise ausgewählt aus Ethylenglykol, 1,2-Propandiol, 1,3-Propandiol, 1,4-Butandiol und Diethylenglykol, und der Alkohol, der mindestens drei Hydroxygruppen aufweist, ist vorzugsweise ausgewählt aus Trimethylolethan, Trimethylolpropan, Trimethylolbutan und Glyzerin.

In dieser Ausführungsform enthält die Komponente (b) vorzugsweise mindestens 25 Gew.-%, mehr bevorzugt mindestens 50 Gew.-% und besonders bevorzugt mindestens 60 Gew.-% und ganz besonders bevorzugt mindestens 75 Gew.-% mindestens eines Alkohols, der mindestens drei Hydroxygruppen aufweist, bezogen auf das Gesamtgewicht der Komponente (b) im Reaktionsgemisch (RG).

Ganz besonders bevorzugt ist der mindestens eine Alkohol, der mindestens zwei Hydroxygruppen aufweist, Ethylenglykol, und der Alkohol, der mindestens drei Hydroxygruppen aufweist, Trimethylolpropan.

Das molare Verhältnis von Komponente (a) zu Komponente (b) beträgt vorzugsweise 20 : 1 bis 5 : 1, bevorzugt 15 : 1 bis 7 : 1 und ganz besonders bevorzugt 12 : 1 bis 9 : 1, bezogen auf die Gesamtstoffmenge der Komponenten (a) und (b) im Reaktionsgemisch (RG).

Vorzugsweise ist die Komponente (b) zumindest teilweise in Komponente (a) gelöst. Die Bezeichnung "zumindest teilweise gelöst" bedeutet dabei, dass vorzugsweise mindestens 5 Gew.-%, mehr bevorzugt mindestens 20 Gew.-% und besonders bevorzugt mindestens 50 Gew.-% der Komponente (b) in Komponente (a) gelöst sind, bezogen auf das Gesamtgewicht der Komponente (b).

Bevorzugt sind mindestens 80 Gew.-%, besonders bevorzugt mindestens 90 Gew.-% der Komponente (b) in der Komponente (a) gelöst, bezogen auf das Gesamtgewicht der Komponente (b) im Reaktionsgemisch (RG). Ganz besonders bevozugt ist die Komponente (b) im Reaktionsgemisch (RG) vollständig in der Komponente (a) gelöst. Dies bedeutet, dass das Reaktionsgemisch (RG) bevorzugt keine separaten Phasen der Komponente (a) und der Komponente (b) aufweist.

Das Lösen der Komponenten (a) und (b) kann durch alle dem Fachmann bekannten Methoden erfolgen. Vorzugsweise erfolgt das Lösen der Komponenten (a) und (b) unter Rühren.

Das Lösen der Komponenten (a) und (b) kann prinzipiell bei beliebigen Temperaturen erfolgen. Vorzugsweise erfolgt das Lösen der Komponenten (a) und (b) bei einer Temperatur im Bereich von 0 bis 30 °C.

Die Komponenten (a) und (b) reagieren im erfindungsgemäßen Verfahren miteinander vorzugsweise in einer Additionsreaktion.

Die Umsetzung des Reaktionsgemisches (RG) kann prinzipiell bei beliebigen Temperaturen erfolgen. Vorzugsweise erfolgt die Umsetzung des Reaktionsgemisches (RG) bei einer Temperatur im Bereich von 20 bis 90 °C, mehr bevorzugt im Bereich von 40 bis 85 °C, besonders bevorzugt im Bereich von 50 bis 80 °C und ganz besonders bevorzugt im Bereich von 60 bis 75 °C. Bei einer Reaktionstemperatur unterhalb von 90°C kann die Bildung von ungewünschten Nebenprodukten neben dem Polyisocyanat (P) verringert oder weitestgehend vermieden werden.

Sämtliche nachfolgenden Angaben bezüglich des Reaktionsgemisches (RG) beziehen sich auf das Gemisch vor der Umsetzung.

Das Reaktionsgemisch (RG) enthält vorzugsweise 70 bis 99 Gew.-% der Komponente (a) und 1 bis 30 Gew.-% der Komponente (b), bezogen auf das Gesamtgewicht des Reaktionsgemisches (RG).

Das Reaktionsgemisch (RG) enthält besonders bevorzugt 80 bis 95 Gew.-% der Komponente (a) und 5 bis 20 Gew.-% der Komponente (b) und ganz besonders bevorzugt 85 bis 92 Gew.-% der Komponente (a) und 8 bis 15 Gew.-% der Komponente (b), bezogen auf das Gesamtgewicht der Komponenten (a) und (b) im Reaktionsgemisch (RG).

Die Umsetzung des Reaktionsgemisches (RG) erfolgt vorzugsweise in einer Inertgasatmosphäre, besonders bevorzugt in einer Stickstoffatmosphäre.

Die Umsetzung des Reaktionsgemisches (RG) kann in Gegenwart mindestens eines Katalysators durchgeführt werden. Bevorzugt erfolgt die Umsetzung des Reaktionsgemisches (RG) allerdings in Abwesenheit eines Katalysators.

Für den Fall, dass die Umsetzung des Reaktionsgemisches (RG) in Gegenwart mindestens eines Katalysators durchgeführt wird, werden die Reaktionsbedingungen der Umsetzung vorzugsweise so gewählt, dass die Bildung von Reaktionsprodukten mit weiteren funktionellen Gruppen wie Allophanat-, Biuret-, Uretdion- oder Isocyanuratgruppen, insbesondere Allophanatgruppen, weitestgehend vermieden wird.

Die Umsetzung des Reaktionsgemisches (RG) in Gegenwart mindestens eines Katalysators erfolgt deshalb bevorzugt bei einer Temperatur, die 90 °C, mehr bevorzugt 85 °C und besonders bevorzugt 80 °C und ganz besonders bevorzugt 75 °C nicht übersteigt.

Darüber hinaus wird vorzugsweise die Umsetzung des Reaktionsgemisches (RG) beendet, vorzugsweise durch Abkühlen des Reaktionsgemisches (RG) auf eine Temperatur unterhalb von 60 °C, mehr bevorzugt unterhalb von 50 °C und besonders bevorzugt unterhalb von 40 °C, sobald mindestens eine der Komponenten (a) oder (b), bevorzugt Komponente (b), vollständig umgesetzt wurde, sodass sich Folgeprodukte, die mit Urethangruppen reagieren, wie insbesondere Allophanatgruppen, nicht bilden.

Als Katalysatoren werden im Rahmen der vorliegenden Erfindung solche Verbindungen verstanden, die eine chemische Reaktion herbeiführen oder beeinflussen, dabei selbst aber nicht verbraucht werden. Geeignete Katalysatoren sind dem Fachmann grundsätzlich bekannt.

Geeignete Katalysatoren umfassen beispielsweise organische Amine, insbesondere tertiäre aliphatische, cycloaliphatische oder aromatische Amine, Brønsted-Säuren und/oder Lewis-saure Metallverbindungen, besonders bevorzugt sind Lewis-saure Organometallverbind ungen.

Als tertiäre aliphatische Amine eignen sich beispielsweise Triethylamin, N,N,N',N'-Tetramethylethylendiamin, N,N,N',N'-Tetramethylpropylendiamin, N,N,N',N",N"-Pentamethyldiethylentriamin, N,N,N',N",N"-Pentamethyl-(3-aminopropyl)ethylendiamin, N,N,N',N',N"-Pentamethyldipropylentriamin, N,N,N',N'-Tetramethylguanidin, N,N,N',N'-Tetramethylhexamethylendiamin, N,N-Dimethylethanolamin, bis(2-Dimethylaminoethyl)ether oder Gemische davon.

Geeignete cycloaliphatische Amine umfassen beispielsweise Triethylendiamin (1,4-Diazabicyclo[2.2.2]octan, DABCO), 1,8-Diaza-bicyclo[5.4.0]undec-7-en (DBU), 1,5-Diazabicyclo-[4.3.0]-non-5-en (DBN), N-Methyl-N'-(2-dimethylaminoethyl)piperazin, N,N'-Dimethylpiperazin, N,N-Dimethylcyclohexylamin, N-Methylmorpholin, N-Ethylmorpholin oder Gemische davon.

Aromatische Amine, die sich als Katalysator eignen sind beispielsweise 1-Methylimidazol, 1,2-Dimethylimidazol, 1-Isobutyl-2-methylimidazol, 1-(N,N-Dimethylaminopropyl)imidazol oder Gemische davon.

Bevorzugte organische Amine sind N,N,N',N'-Tetramethylethylendiamin, N,N,N',N'-Tetramethylpropylendiamin, N,N,N',N'-Tetramethylguanidin, Triethylendiamin (1,4-Diazabicyclo[2.2.2]octan, DABCO), 1,8-Diaza-bicyclo[5.4.0]undec-7-en (DBU), 1,5-Diazabicyclo-[4.3.0]-non-5-en (DBN), N,N'-Dimethylpiperazin oder Gemische davon.

Geeignete Brønsted-Säuren umfassen beispielsweise Trifluoressigsäure, Schwefelsäure, Chlorwasserstoff, Bromwasserstoff, Phosphorsäure, Perchlorsäure oder Gemische davon.

Geeignete Lewis-saure Organometallverbindungen sind insbesondere Zinkverbindungen und Zinnverbindungen, besonders bevorzugt solche von Carbonsäuren, die mindestens zwei und bis zu zwölf Kohlenstoffatome aufweisen. Entsprechende Zinkverbindungen umfassen beispielsweise Zink(II)-stearat, Zink(II)-n-octanoat, Zink(II)-2-ethylhexanoat, Zink(II)-naphthenat, Zink(II)-acetylacetonat oder Gemische davon. Ein handelsüblicher Zink-haltiger Katalysator ist beispielsweise Borchi® Kat 22 von OMG Borchers GmbH, Langenfeld, Deutschland.

Zinnverbindungen umfassen beispielsweise Zinn(II)-acetat, Zinn(II)-n-octanoat, Zinn(II)-2-ethylhexanoat, Zinn(II)-laurat, Dibutylzinn(IV)-oxid, Dibutylzinn(IV)-chlorid, Dibutylzinn(IV)-acetat, Dibutylzinn(IV)-butyrat, Dibutylzinn(IV)-2-ethylhexanoat, Dibutylzinn(IV)-laurat, Dibutylzinn(IV)-maleat, Dioctylzinn(IV)-acetat, Dioctylzinn(IV)-laurat oder Gemische davon.

Weitere Lewis-saure Verbindungen umfassen Metallhalogenide und Metallkomplexe, vorzugsweise Acetylacetonatokomplexe und sonstige Dionatokomplexe, des Eisens, Aluminiums, Zirkoniums, Mangans, Nickels und Cobalts oder Gemische davon. Besonders bevorzugte Metallkomplexe sind Zirkonium(IV)-acetylacetonat und sonstige Zirkonium(IV)-dionate, sowie Aluminum(III)-dionate, die handelsüblich erhältlich sind unter den Bezeichnungen K-KAT® 4205, K-KAT® XC-9213; K-KAT® XC-A 209, K-KAT® XC-6212 und K-KAT® 5218 der Firma King Industries. Weitere Metallkatalysatoren werden von Blank et al. in Progress in Organic Coatings, 1999, Vol. 35, Seiten 19 bis 29 beschrieben.

Als Zinn- und Zink-freie Alternativen eignen sich außerdem Cäsium-, Bismut-, und Titan-Verbindungen.

Als Cäsium- und Bismutverbindungen kommen dabei solche in Betracht, in denen folgende Anionen eingesetzt werden: F⁻, Cl⁻, ClO⁻, ClO₃⁻, ClO₄⁻, Br, I⁻, IO₃⁻, CN-, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₃²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄^{3-,} P₂O₇⁴⁻, (OCₙH₂ₙ₊₁)⁻, (CₙH₂ₙ₋₁O₂)⁻, (CₙH₂ₙ₋₃O₂)⁻ oder (Cₙ₊₁H₂ₙ₋₂O₄)²⁻, wobei n für die Zahlen 1 bis 20 steht. Bevorzugte Anionen sind Anionen der Formeln (CₙH₂ₙ₋₁O₂)⁻ oder (Cₙ₊₁H₂ₙ₋₂O₄)²⁻, wobei n für die Zahlen 1 bis 20 steht, besonders bevorzugt Anionen der Formel (CₙH₂ₙ₋₁O₂)⁻, wobei n für die Zahlen 1 bis 20 steht.

Bevorzugte Cäsium- und Bismutverbindungen sind insbesondere Cäsium(I)-Formiat, Cäsium(I)-Acetat, Cäsium(I)-n-Propionat, Cäsium(I)-n-Hexanoat, Cäsium(I)-neodekanoat, Cäsium(I)-2-Ethylhexanoat, Bismut(III)-octanoat, Bismut(III)-2-Ethylhexanoat, Bismut(III)-neodecanoat oder Bismut(III)-pivalat oder Gemische davon.

Entsprechende handelsübliche Cäsium- und Bismut-haltige Katalysatoren sind beispielsweise K-KAT 348, XC-B221; XC-C227, XC 8203 und XK-601 von King Industries, TIB KAT 716, 716LA, 716XLA, 718, 720, 789 von TIB Chemicals sowie beispielsweise Borchi® Kat 24, Borchi® Kat 315 oder Borchi® Kat 320 von OMG Borchers GmbH, Langenfeld, Deutschland.

Unter den Titanverbindungen sind Titan(IV)-alkoholate Ti(OR)₄ bevorzugt, besonders bevorzugt solche von Alkoholen ROH mit 1 bis 8 Kohlenstoffatomen, beispielsweise Methanol, Ethanol, Isopropanol, n-Propanol, n-Butanol, Isobutanol, sec-Butanol, tert-Butanol, n-Hexanol, n-Heptanol, n-Octanol, bevorzugt sind Methanol, Ethanol, Isopropanol, n-Propanol, n-Butanol oder tert-Butanol, besonders bevorzugt sind Isopropanol oder n-Butanol.

Der mindestens eine Katalysator wird je nach Aktivität normalerweise in Mengen von 0,001 bis 10 mol-%, bezogen auf die Gesamtstoffmenge an Isocyanatgruppen der Komponente (a), eingesetzt.

Die Umsetzung des Reaktionsgemisches (RG) wird bevorzugt ohne Lösungsmittel durchgeführt, kann aber auch in Gegenwart mindestens eines Lösungsmittels durchgeführt werden. Geeignete Lösungsmittel sind dem Fachmann im Allgemeinen bekannt.

Als Lösungsmittel können im Allgemeinen alle Lösungsmittel verwendet werden, die keine gegenüber Isocyanatgruppen reaktiven Gruppen aufweisen. Beispiele für derartige Lösungsmittel sind aromatische, aliphatische und/oder cycloaliphatische Kohlenwasserstoffe und deren Gemische, chlorierte Kohlenwasserstoffe, Ketone, Ester, alkoxylierte Alkansäurealkylester, Ether sowie Gemische der vorstehend genannten Lösungsmittel.

Durch Umsetzung des Reaktionsgemisches (RG) wird das Polyisocyanat (P) gemäß den vorstehenden Definitionen erhalten.

In einer bevorzugten Ausführungsform wird die Umsetzung des Reaktionsgemisches (RG) abgebrochen, nachdem mindestens ein Teil der Komponenten (a) und (b) umgesetzt wurde. Vorzugsweise wird die Umsetzung des Reaktionsgemisches (RG) erst abgebrochen, nachdem mindestens eine der Komponenten (a) oder (b), vorzugsweise Komponente (b), vollständig umgesetzt wurde. Der Abbruch der Umsetzung des Reaktionsgemisches (RG) kann durch beliebige dem Fachmann bekannte Methoden erfolgen.

Vorzugsweise wird die Umsetzung des Reaktionsgemisches (RG) durch Zugabe mindestens einer Komponente (c) abgebrochen, nachdem mindestens ein Teil der Komponenten (a) und (b) umgesetzt wurde. Die mindestens eine Komponente (c) verhindert eine Weiterreaktion des Polyisocyanats (P) mit weiteren Molekülen des Polyisocyanats (P) oder mit sonstigen im Reaktionsgemisch (RG) enthaltenen Verbindungen und sorgt somit für eine erhöhte Produktreinheit. Insbesondere führt der Einsatz der mindestens einen Komponente (c) dazu, dass die Bildung von Verbindungen mit Allophanatgruppen oder Isocyanuratgruppen weitestgehend vermieden werden kann.

Geeignete Komponenten (c) umfassen beispielsweise organische Säuren bzw. Säurechloride wie Benzolsulfonsäure, Benzolsulfonsäurechlorid, Toluolsulfonsäure, Toluolsulfonsäurechlorid, Benzoesäure, Benzoylchlorid, Benzylchlorid, phosphorige Säure, Phosphorsäure oder saure Ester der phosphorigen Säure oder der Phosphorsäure wie beispielsweise Dibutylphosphit, Dibutylphosphat oder Di(2-ethylhexyl)phosphat.

Vorzugsweise ist die mindestens eine Komponente (c) ausgewählt aus Toluolsulfonsäure, Toluolsulfonsäurechlorid, Benzoylchlorid, Benzylchlorid, Dibutylphosphit, Dibutylphosphat oder Di(2-ethylhexyl)phosphat.

Vorzugsweise werden noch nicht umgesetzte Mengen an Komponente (a) und Komponente (b) vom Polyisocyanat (P) abgetrennt. Die Abtrennung kann dabei durch alle dem Fachmann bekannten Methoden erfolgen, beispielsweise durch Dünnschichtdestillation, Extraktion, Kristallisation oder Molekulardestillation. Besonders bevorzugt erfolgt die Abtrennung mittels Dünnschichtdestillation.

Die Abtrennung der Komponenten (a) und (b) vom Polyisocyanat (P) erfolgt vorzugsweise unter Verwendung von inerten protischen oder aprotischen Lösungsmitteln. Geeignete Lösungsmittel wurden unter anderem vorstehend bereits beschrieben und sind solche, die keine gegenüber Isocyanatgruppen reaktiven Gruppen aufweisen.

Das durch das erfindungsgemäße Verfahren hergestellte Polyisocyanat (P) eignet sich in vielen technischen Bereichen, beispielsweise als Vernetzungsreagenz in Klarlacken, Klebstoffen, Beschichtungsmassen und Automobillacken, bevorzugt als Vernetzungsreagenz in Klarlacken.

Ein weiterer Gegenstand der vorliegenden Erfindung ist somit auch die Verwendung des Polyisocyanats (P) als Vernetzungsreagenz in Klarlacken.

Die folgenden Beispiele sollen die vorliegende Erfindung weiter verdeutlichen, ohne aber die vorliegende Erfindung darauf zu beschränken.

### Beispiele

Als das mindestens eine zyklische Isocyanat (a) (Komponente (a)) wird in den nachfolgenden Ausführungsbeispielen MCDI (ein Gemisch aus 1,3-Diiso-cyanato-4-methyl-cyclohexan und 1,3-Diisocyanato-2-methylcyclohexan) verwendet. Als Alkohol, der zwei Hydroxygruppen aufweist, wird in den nachfolgenden Beispielen Diethylenglykol verwendet. Als Alkohol, der mindestens drei Hydroxygruppen aufweist, wird in den nachfolgenden Beispielen Trimethylolpropan eingesetzt.

Die Bestimmung des Gehalts an NCO-Gruppen in Polyisocyanaten erfolgt durch die nachfolgend beschriebene Methode:
0,5 g eines polyisocyanathaltigen Gemisches werden genau eingewogen und in 100 ml N-Methylpyrrolidon gelöst. Zu dieser Lösung werden 10 ml eine 1 N Lösung von n-Dibutylamin in Xylol gegeben. Das so erhaltene Gemisch wird bei Raumtemperatur für 15 Minuten stehen gelassen, wobei das Polyisocyanat und n-Dibutylamin miteinander reagieren. Nach vollständiger Reaktion wird das entstandene Gemisch mit 1 N Salzsäure rücktitriert, um das Gesamtvolumen an Salzsäure, welches zur Neutralisation von unreagiertem n-Dibutylamin im Gemisch benötigt wird, zu bestimmen.

Die Charakterisierung der durch das erfindungsgemäße Verfahren erhaltenen Polyisocyanate erfolgt mittels Gelpermeationschromatographie (GPC).

### Gerätebeschreibung der GPC-Anlage:

| | |
|---|---|
| Injektor: | Autosampler WATERS 717 Plus |
| Eluent: | Tetrahydrofuran (Flussrate: 0,5 ml/min) |
| Pumpe: | WATERS Modell 515 (Doppelkolbenpumpe) |
| Detektor 1: | UV-Detektor WATERS 2489 (Wellenlänge: 254 nm) |
| Detektor 2: | Differentialrefraktometer WATERS 2414 (Messtemperatur: 35°C) |
| Säulensatz: | PL-Gel-Säulen (600x7,5 mm), 4 Säulen in Reihe geschaltet |
| Säulenmaterial: | vernetzte Polystyrol-Divinylbenzol-Matrix, Teilchengröße 5 µm |
| Porenweite: | 1 x 50 Å |
| | 3 x 100 Å |
| Kalibration: | Basonat HI 100, Molmassenbereich 2200 - 168 g/mol |
| Software: | PSS WinGPC Unity NT |

### Vergleichsbeispiel 1

500 g Isophorondiisocyanat (IPDI), 44 g Trimethylolpropan und 14.16 g Diethylenglykol werden in einem 1 I Dreihalskolben mit Thermometer (verbunden mit einem Ölbad mit einstellbarer Temperatur), Rührer, Rückflusskühler und Stickstoffeinlass vorgelegt und unter Rühren auf 80 °C erhitzt. Die Veränderung des NCO-Gehalts während der Umsetzung der Ausgangsmaterialien wird wie vorstehend ausgeführt durch Titration bestimmt. Die Reaktion wird durch Zugabe von 300 ppm eines Gemisches aus Benzylchlorid und bis(2-ethylhexyl)phosphat (50/50 Gew.-%) abgebrochen, nachdem der NCO-Gehalt den theoretischen Wert für die vollständige Umsetzung des Isophorondiisocyanats mit Trimethylolpropan und Diethylenglykol erreicht hat. Die so erhaltene farblose Zusammensetzung wird bei 3 mbar und 175 °C mittels Dünnschichtdestillation aufgereinigt, um nicht umgesetztes Isophorondiisocyanat abzutrennen.

240,1 g dieser Zusammensetzung werden in 102,9 g Butylacetat gelöst. Die resultierende gelbliche Lösung (70 Gew.-% in Butylacetat) weist einen NCO-Gehalt von 8,6 % und eine Viskosität von 1810 mPa · s auf.

### Ausführungsbeispiel 1

500 g MCDI, 44 g Trimethylolpropan und 14.16 g Diethylenglykol werden in einem 1 I Dreihalskolben mit Thermometer (verbunden mit einem Ölbad mit einstellbarer Temperatur), Rührer, Rückflusskühler und Stickstoffeinlass vorgelegt und unter Rühren auf 80 °C erhitzt. Die Veränderung des NCO-Gehalts während der Umsetzung der Ausgangsmaterialien wird wie vorstehend ausgeführt durch Titration bestimmt. Die Reaktion wird durch Zugabe von 300 ppm eines Gemisches aus Benzylchlorid und bis(2-ethylhexyl)phosphat (50/50 Gew.-%) abgebrochen, nachdem der NCO-Gehalt den theoretischen Wert für die vollständige Umsetzung des MCDI mit Trimethylolpropan und Diethylenglykol erreicht hat. Die so erhaltene farblose Zusammensetzung wird bei 3 mbar und 175 °C mittels Dünnschichtdestillation aufgereinigt, um nicht umgesetztes Isophorondiisocyanat abzutrennen.

251,1 g dieser Zusammensetzung werden in 107,6 g Butylacetat gelöst. Die resultierende gelbliche Lösung (70 Gew.-% in Butylacetat) weist einen NCO-Gehalt von 10,6 % und eine Viskosität von 2000 mPa · s auf.

## Patentansprüche

1. Polyisocyanat (P) der allgemeinen Formel (I): 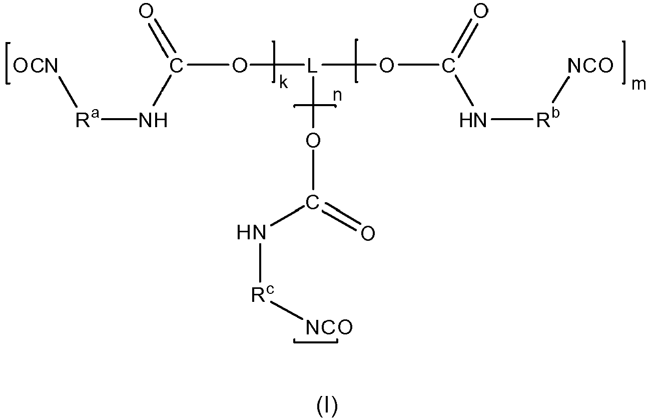 in der
k, m, n unabhängig voneinander 0, 1, 2 oder 3 sind, wobei die Summe von k, m und n mindestens 2 und höchstens 6 ergibt; und
L ein linearer oder verzweigter organischer Rest mit höchstens 14 Kohlenstoffatomen ist, der gegebenenfalls mindestens ein Stickstoffatom und/oder mindestens ein Sauerstoffatom enthalten kann,
R^{a}, R^{b}, R^{c} unabhängig voneinander ausgewählt sind aus Resten der allgemeinen Formeln (IIa), (IIb), (IIc), (IId), (IIe) und/oder (IIf): 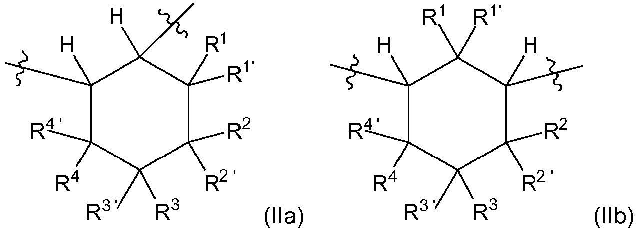 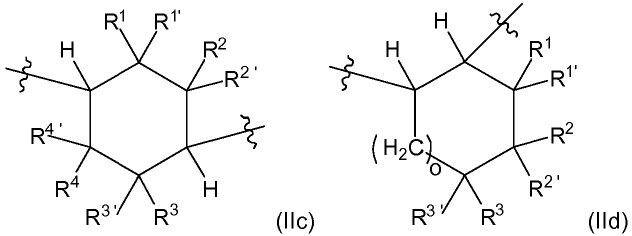 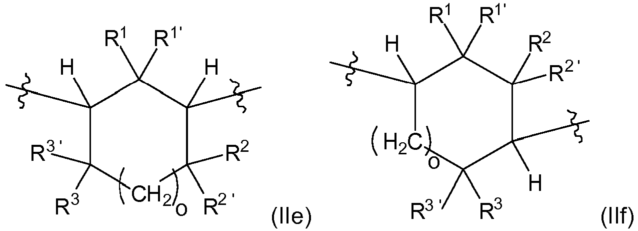
in denen
o 0 bis 10 ist, und
R¹, R^{1'}, R², R^{2'}, R³, R^{3'}, R⁴, R^{4'} unabhängig voneinander ausgewählt sind aus der Gruppe bestehend aus H, OR⁵ und unsubstituiertem oder zumindest monosubstituiertem C₁-C₁₀-Alkyl, C₅-C₁₂-Cycloalkyl, C₂-C₁₀-Alkenyl und C₆-C₁₄-Aryl, wobei R⁵ C₁-C₁₀-Alkyl ist.

2. Polyisocyanat (P) gemäß Anspruch 1, **dadurch gekennzeichnet, dass**
k, m, n unabhängig voneinander 0, 1, 2 oder 3 sind, wobei die Summe von k, m und n mindestens 2 und höchstens 6 ergibt; und
L ein linearer oder verzweigter aliphatischer, cycloaliphatischer oder aromatischer Rest mit höchstens 14 Kohlenstoffatomen ist, der gegebenenfalls mindestens ein Stickstoffatom und/oder mindestens ein Sauerstoffatom enthalten kann,
R^{a}, R^{b}, R^{c} unabhängig voneinander ausgewählt sind aus Resten der allgemeinen Formeln (IIa), (IIb), (IIc), (IId), (IIe) und/oder (IIf): 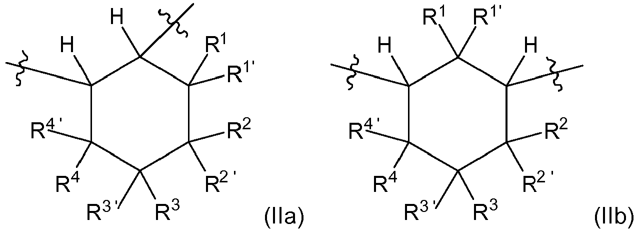 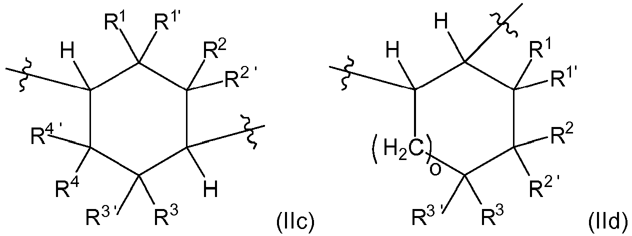 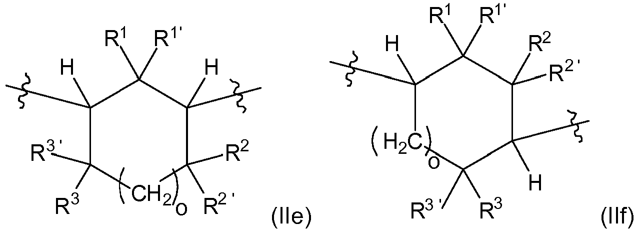
in denen
o 0, 2 oder 3 ist, und
R¹, R^{1'}, R², R^{2'}, R³, R^{3'}, R⁴, R^{4'} unabhängig voneinander ausgewählt sind aus der Gruppe bestehend aus H und C₁-C₁₀-Alkyl.

3. Polyisocyanat (P) gemäß Anspruch 1 oder 2, **dadurch gekennzeichnet, dass**
k, m, n unabhängig voneinander 0, 1, 2 oder 3 sind, wobei die Summe von k, m und n mindestens 2 und höchstens 6 ergibt; und
L ein linearer oder verzweigter aliphatischer oder cycloaliphatischer Rest mit höchstens 14 Kohlenstoffatomen ist, der gegebenenfalls mindestens ein Stickstoffatom und/oder mindestens ein Sauerstoffatom enthalten kann,
R^{a}, R^{b}, R^{c} unabhängig voneinander ausgewählt sind aus Resten der allgemeinen Formeln (IIa), (IIb) und/oder (IIc): 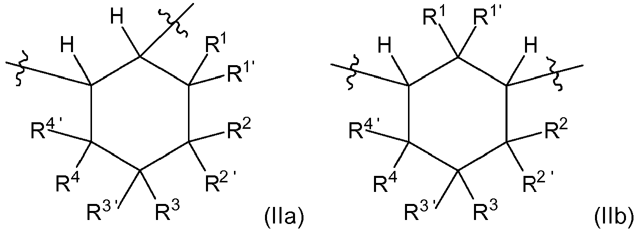 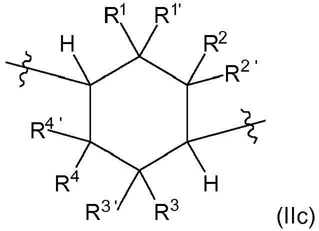
in denen
R¹, R^{1'}, R², R^{2'}, R³, R^{3'}, R⁴, R^{4'} unabhängig voneinander ausgewählt sind aus der Gruppe bestehend aus H, Methyl, Ethyl und Propyl.

4. Polyisocyanat (P) gemäß einem der Ansprüche 1 bis 3, **dadurch gekennzeichnet, dass**
k 2 oder 3 ist, und
m, n 0 sind, und
L ein linearer oder verzweigter aliphatischer Rest mit höchstens 10 Kohlenstoffatomen ist, der gegebenenfalls mindestens ein Stickstoffatom und/oder mindestens ein Sauerstoffatom enthalten kann,
R^{a} ausgewählt ist aus Resten der allgemeinen Formeln (IIa), (IIb) und/oder (IIc): 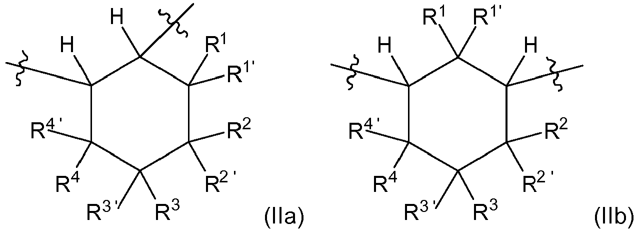 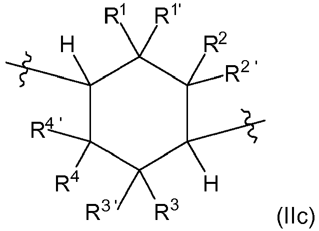
in denen
R¹, R^{1'}, R², R^{2'}, R³, R^{3'}, R⁴, R^{4'} unabhängig voneinander ausgewählt sind aus der Gruppe bestehend aus H, Methyl und Ethyl,
wobei mindestens ein Rest R¹, R¹', R², R^{2'}, R³, R^{3'}, R⁴ oder R^{4'} Methyl oder Ethyl, vorzugsweise Methyl, ist.

5. Polyisocyanat (P) gemäß einem der Ansprüche 1 bis 4, **dadurch gekennzeichnet, dass**
k 3 ist, und
m, n 0 sind, und
L ein linearer oder verzweigter aliphatischer Rest mit höchstens 6 Kohlenstoffatomen ist, der gegebenenfalls mindestens ein Sauerstoffatom enthalten kann,
R^{a} ein Rest der allgemeinen Formel (IIb) ist: 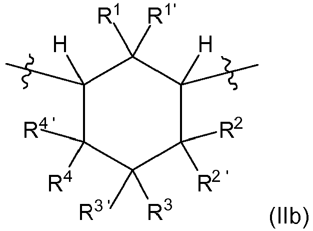
in der
R¹, R^{1'}, R², R^{2'}, R³, R^{3'}, R⁴, R^{4'} unabhängig voneinander ausgewählt sind aus der Gruppe bestehend aus H, Methyl und Ethyl,
wobei mindestens ein Rest und höchstens 3 Reste R¹, R^{1'}, R², R^{2'}, R³, R^{3'}, R⁴ oder R^{4'} Methyl oder Ethyl, vorzugsweise Methyl, sind.

6. Polyisocyanat (P) der allgemeinen Formel (IV) 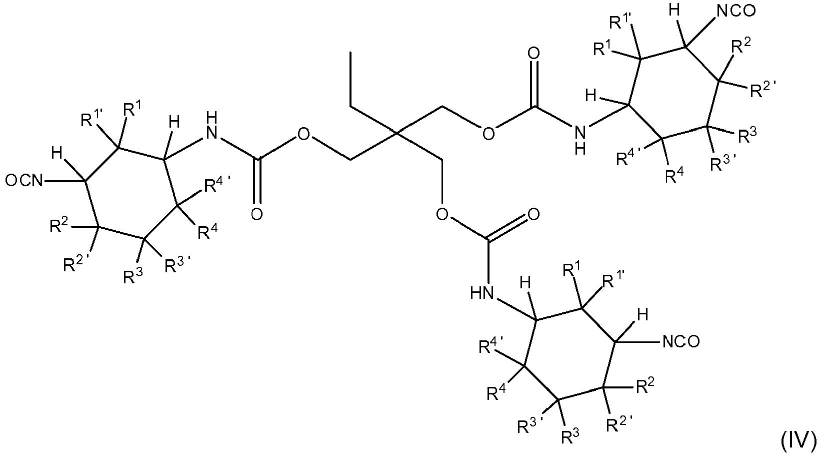 in der
R¹, R¹', R², R^{2'} unabhängig voneinander ausgewählt sind aus der Gruppe bestehend aus H und Methyl, wobei mindestens ein Rest und höchstens zwei Reste R¹, R¹', R² oder R^{2'} Methyl sind, und
R³, R^{3'}, R⁴, R^{4'} H sind.

7. Verfahren zur Herstellung des Polyisocyanats (P) gemäß einem der Ansprüche 1 bis 6, umfassend die Umsetzung eines Reaktionsgemisches (RG) enthaltend die folgenden Komponenten (a) und (b):
(a) mindestens ein zyklisches Isocyanat der allgemeinen Formeln (IIIa), (IIIb), (IIIc), (IIId), (IIIe) und/oder (IIIf): 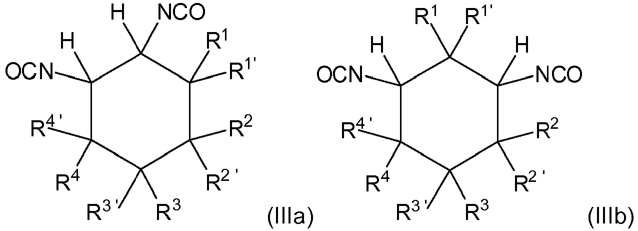 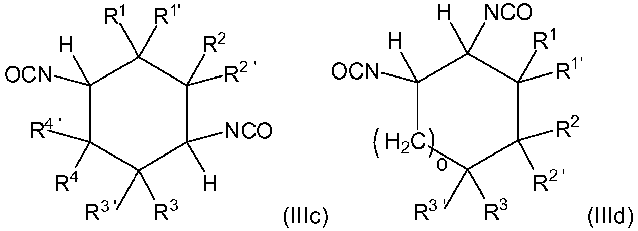 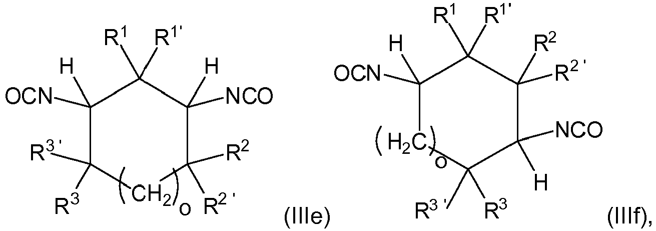 in denen
o 0 bis 10 ist, und
R¹, R^{1'}, R², R^{2'}, R³, R^{3'}, R⁴, R^{4'} unabhängig voneinander ausgewählt sind aus der Gruppe bestehend aus H, OR⁵ und unsubstituiertem oder zumindest monosubstituiertem C₁-C₁₀-Alkyl, C₅-C₁₂-Cycloalkyl, C₂-C₁₀-Alkenyl und C₆-C₁₄-Aryl, wobei R⁵ C₁-C₁₀-Alkyl ist, und
(b) mindestens ein Alkohol, der mindestens zwei Hydroxygruppen aufweist,
unter Erhalt des Polyisocyanats (P).

8. Verfahren gemäß Anspruch 7, **dadurch gekennzeichnet, dass** die Komponente (a) ausgewählt ist aus 1,3-Diisocyanatocyclohexan, 1,3-Diisocyanato-2-methylcyclohexan, 1,3-Diisocyanato-4-methylcyclohexan, 1,3-Diisocyanato-5-methylcyclohexan, 1,3-Diisocyanato-2-isopropylcyclohexan, 1,3-Diisocyanato-4-isopropylcylohexan, 3-Diisocyanato-5-isopropylcyclohexan, 1,3-Diisocyanato-2,4-dimethylcyclohexan, 1,3-Diisocyanato-2,4-diethylcyclohexan, 1,3-Diiso-cyanato-2,4-diethyl-6-methylcyclohexan, 1,3-Diisocyanato-2-methyl-4,5-diethyl-cyclohexan, 1,3-Diisocyanato-2,4,6-triisopropylcyclohexan oder 1,3-Diisocyanato-2,4,6-tributylcyclohexan.

9. Verfahren gemäß Anspruch 7 oder 8, **dadurch gekennzeichnet, dass** die Komponente (b) ausgewählt ist aus Ethylenglykol, 1,1-Dimethylethylenglykol, 1,2-Propandiol, 1,3-Propandiol, 2-Methyl-1,3-Propandiol, 2-Ethyl-1,3-Propandiol, 2-Butyl-2-ethyl-1,3-Propandiol, 1,4-Butandiol, 2-Ethyl-1,4-butandiol, 1,5-Pentandiol, 2-Methyl-1,5-pentandiol, Neopentylglykol, 1,6-Hexandiol, 2-Ethyl-1,3-Hexandiol, 2,4-Diethyloktan-1,3-diol, Diethylenglykol, Triethylenglykol, Tetraethylenglykol, Pentaethylenglykol, Dipropylenglykol, Tripropylenglykol, Trimethylolethan, Trimethylolpropan, Trimethylolbutan, Ditrimethylolpropan, Pentaerythrit, Dipentaerythrit, Glyzerin, Cyclohexan-1,2-diol, Cyclohexan-1,3-diol, Cyclohexan-1,4-diol, 1,1-Bis(hydroxymethyl)cyclohexan, 1,2-Bis(hydroxymethyl)-cyclohexan, 1,3-Bis(hydroxymethyl)cyclohexan, 1,4-Bis(hydroxymethyl)cyclohexan, 2,2-Bis(4-hydroxycyclohexyl)propan, Erythrit, Threit, Xylit, Adonit (Ribit), Arabit (Lyxit), Sorbit, Mannit, Dulcit (Galactit), Maltit, Isomalt, Diglyzerin, Dimethylolpropan, Dipentaerythritol, Ribose, Arabinose, Glucose, Mannose, Galactose, Fructose, Brenzcatechin, Catechol, Hydrochinon, Pyrogallol, Hydroxyhydrochinon, Phloroglucin, Hydroxypivalinsäureneopentylglykolester, Triethanolamin, Tripropanolamin, 1,3,5-tris(2-Hydroxyethyl)cyanursäure, Polytetrahydrofuran mit einem Molekulargewicht zwischen 162 und 4500 g/mol, bevorzugt 250 bis 2000 g/mol, Poly-1,3-propandiol oder Polypropylenglykol mit einem Molekulargewicht zwischen 134 und 2000 g/mol oder Polyethylenglykol mit einem Molekulargewicht zwischen 238 und 2000 g/mol.

10. Verfahren gemäß einem der Ansprüche 7 bis 9, **dadurch gekennzeichnet, dass** die Komponente (b) mindestens zwei Alkohole umfasst, wobei mindestens ein Alkohol zwei Hydroxygruppen aufweist und mindestens ein Alkohol mindestens drei Hydroxygruppen aufweist.

11. Verfahren gemäß einem der Ansprüche 7 bis 10, **dadurch gekennzeichnet, dass** das Reaktionsgemisch (RG) 70 bis 99 Gew.-% der Komponente (a) und 1 bis 30 Gew.-% der Komponente (b), besonders bevorzugt 80 bis 95 Gew.-% der Komponente (a) und 5 bis 20 Gew.-% der Komponente (b) und ganz besonders bevorzugt 85 bis 92 Gew.-% der Komponente (a) und 8 bis 15 Gew.-% der Komponente (b) enthält, bezogen auf das Gesamtgewicht der Komponenten (a) und (b) im Reaktionsgemisch (RG).

12. Verfahren gemäß einem der Ansprüche 7 bis 11, **dadurch gekennzeichnet, dass**
i) die Umsetzung des Reaktionsgemisches (RG) bei einer Temperatur im Bereich von 20 bis 90 °C, besonders bevorzugt im Bereich von 40 bis 85 °C und ganz besonders bevorzugt im Bereich von 50 bis 80 °C erfolgt, und/oder
ii) die Umsetzung des Reaktionsgemisches (RG) in Abwesenheit eines Katalysators erfolgt.

13. Verfahren gemäß einem der Ansprüche 7 bis 12, **dadurch gekennzeichnet, dass** die Umsetzung des Reaktionsgemisches (RG) durch Zugabe mindestens einer Komponente (c) abgebrochen wird, nachdem mindestens ein Teil der Komponenten (a) und (b) umgesetzt wurde,
wobei die mindestens eine Komponente (c) vorzugsweise ausgewählt ist aus Toluolsulfonsäure, Toluolsulfonsäurechlorid, Benzoylchlorid, Benzylchlorid, Dibutylphosphit, Dibutylphosphat oder Di-(2-ethylhexyl)phosphat.

14. Verfahren gemäß einem der Ansprüche 7 bis 13, **dadurch gekennzeichnet, dass**
i) die Umsetzung abgebrochen wird, nachdem mindestens eine der Komponenten (a) oder (b), vorzugsweise Komponente (b), vollständig umgesetzt wurde, und/oder
ii) noch nicht umgesetzte Mengen an Komponente (a) und/oder (b) aus der Zusammensetzung (Z) abgetrennt werden.

15. Verwendung des Polyisocyanats (P) gemäß einem der Ansprüche 1 bis 6 als Vernetzungsreagenz in Klarlacken.

## Claims

1. A polyisocyanate (P) of general formula (I): 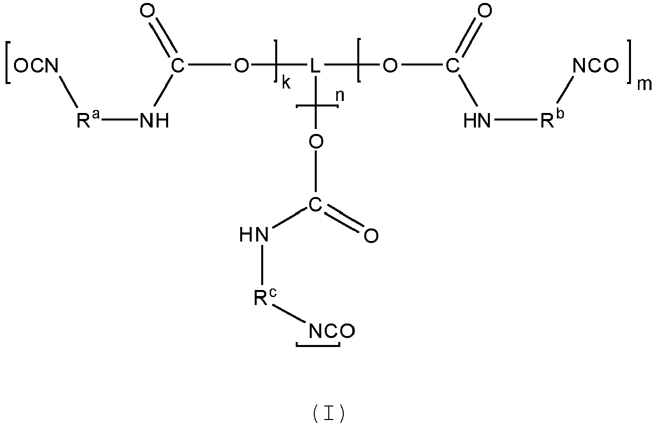 in which
k, m, n are each independently 0, 1, 2 or 3, wherein the sum total of k, m and n is at least 2 and at most 6; and
L is a linear or branched organic radical having at most 14 carbon atoms, which may optionally comprise at least one nitrogen atom and/or at least one oxygen atom,
R^{a}, R^{b}, R^{c} are each independently selected from radicals of general formulae (IIa), (IIb), (IIc), (IId), (IIe) and/or (IIf): 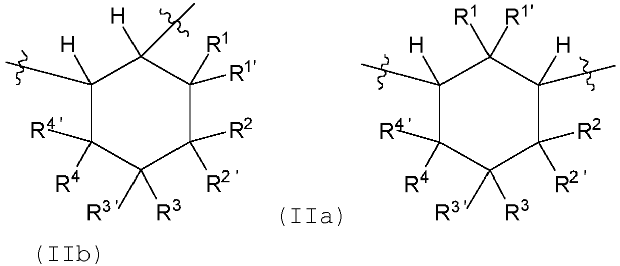 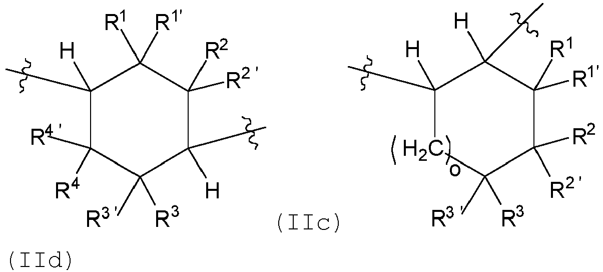 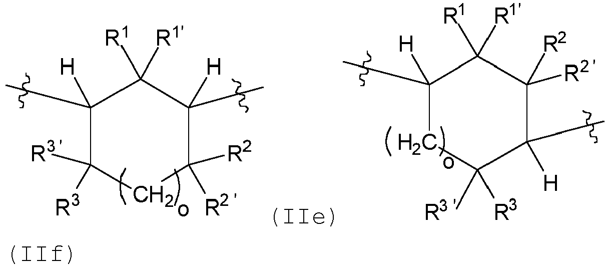
in which
o is 0 to 10, and
R¹, R^{1'}, R², R^{2'}, R³, R^{3'}, R⁴, R^{4'} are each independently selected from the group consisting of H, OR⁵ and unsubstituted or at least monosubstituted C₁-C₁₀-alkyl, C₅-C₁₂-cycloalkyl, C₂-C₁₀-alkenyl and C₆-C₁₄-aryl, where R⁵ is C₁-C₁₀-alkyl.

2. The polyisocyanate (P) according to claim 1, wherein
k, m, n are each independently 0, 1, 2 or 3, wherein the sum total of k, m and n is at least 2 and at most 6; and
L is a linear or branched aliphatic, cycloaliphatic or aromatic radical having at most 14 carbon atoms, which may optionally comprise at least one nitrogen atom and/or at least one oxygen atom,
R^{a}, R^{b}, R^{c} are each independently selected from radicals of general formulae (IIa), (IIb), (IIc), (IId), (IIe) and/or (IIf): 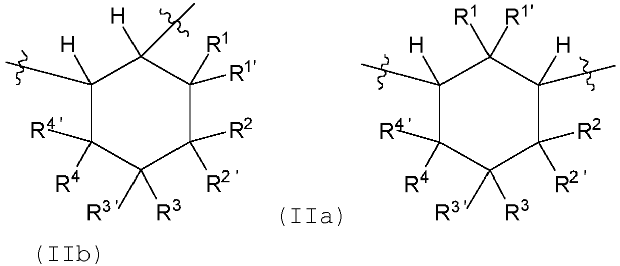 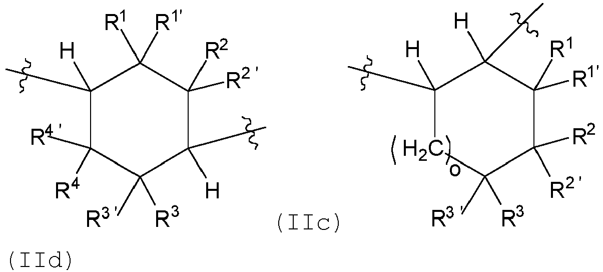 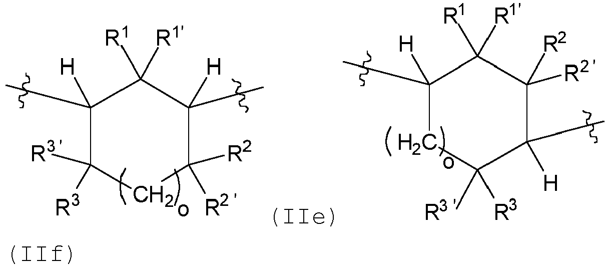
in which
o is 0, 2 or 3, and
R¹, R^{1'}, R², R^{2'}, R³, R^{3'}, R⁴, R^{4'} are each independently selected from the group consisting of H and C₁-C₁₀-alkyl.

3. The polyisocyanate (P) according to claim 1 or 2, wherein
k, m, n are each independently 0, 1, 2 or 3, wherein the sum total of k, m and n is at least 2 and at most 6; and
L is a linear or branched aliphatic or cycloaliphatic radical having at most 14 carbon atoms, which may optionally comprise at least one nitrogen atom and/or at least one oxygen atom,
R^{a}, R^{b}, R^{c} are each independently selected from radicals of general formulae (IIa), (IIb) and/or (IIc): 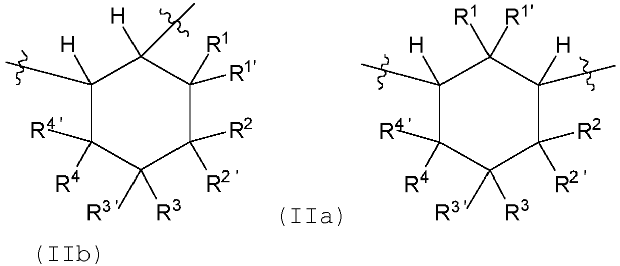 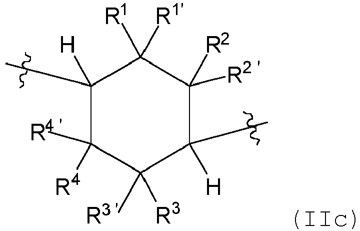
in which
R¹, R^{1'}, R², R^{2'}, R³, R^{3'}, R⁴, R^{4'} are each independently selected from the group consisting of H, methyl, ethyl and propyl.

4. The polyisocyanate (P) according to any of claims 1 to 3, wherein
k is 2 or 3, and
m, n are 0, and
L is a linear or branched aliphatic radical having at most 10 carbon atoms, which may optionally comprise at least one nitrogen atom and/or at least one oxygen atom,
R^{a} is selected from the radicals of general formulae (IIa), (IIb) and/or (IIc): 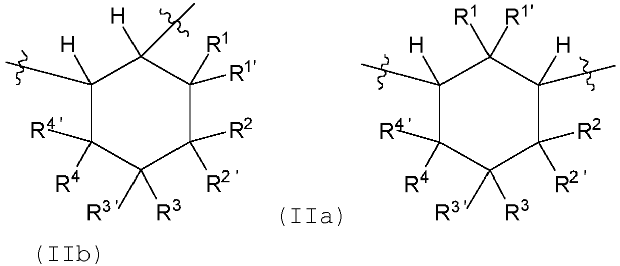 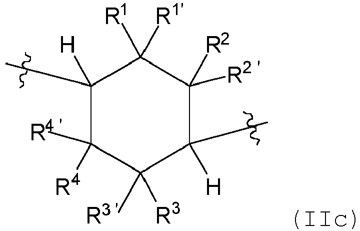
in which
R¹, R^{1'}, R², R^{2'}, R³, R^{3'}, R⁴, R^{4'} are each independently selected from the group consisting of H, methyl and ethyl,
wherein at least one radical R¹, R^{1'}, R², R^{2'}, R³, R^{3'}, R⁴ or R^{4'} is methyl or ethyl, preferably methyl.

5. The polyisocyanate (P) according to any of claims 1 to 4, wherein
k is 3, and
m, n are 0, and
L is a linear or branched aliphatic radical having at most 6 carbon atoms, which may optionally comprise at least one oxygen atom,
R^{a} is a radical of general formula (IIb): 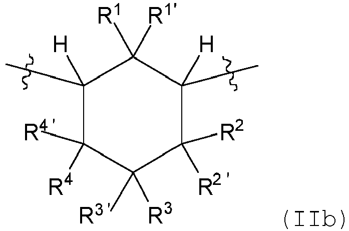
in which
R¹, R^{1'}, R², R^{2'}, R³, R^{3'}, R⁴, R^{4'} are each independently selected from the group consisting of H, methyl and ethyl,
wherein at least one radical and at most 3 radicals R¹, R^{1'}, R², R^{2'}, R³, R^{3'}, R⁴ or R^{4'} are methyl or ethyl, preferably methyl.

6. A polyisocyanate (P) of general formula (IV) 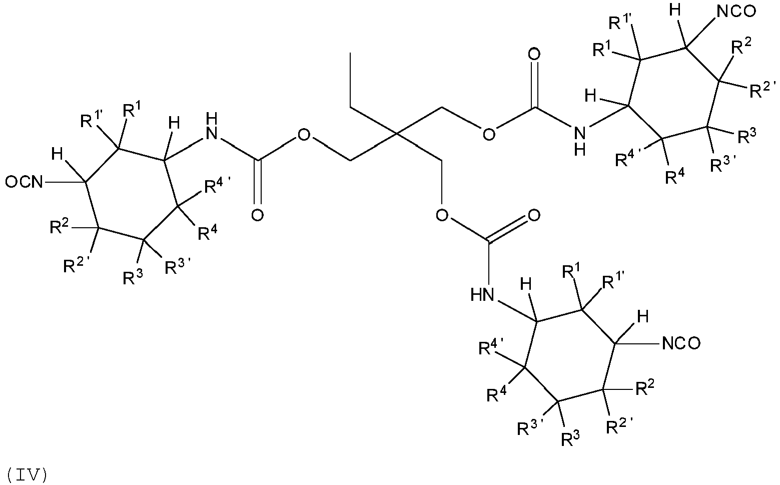 in which
R¹, R^{1'}, R², R^{2'} are each independently selected from the group consisting of H and methyl, wherein at least one radical and at most two radicals R¹, R^{1'}, R² or R^{2'} are methyl, and
R³, R^{3'}, R⁴ and R^{4'} are H.

7. A method for preparing the polyisocyanate (P) according to any of claims 1 to 6, comprising the reaction of a reaction mixture (RM) comprising the following components (a) and (b):
(a) at least one cyclic isocyanate of general formulae (IIIa), (IIIb), (IIIc), (IIId), (IIIe) and/or (IIIf) : 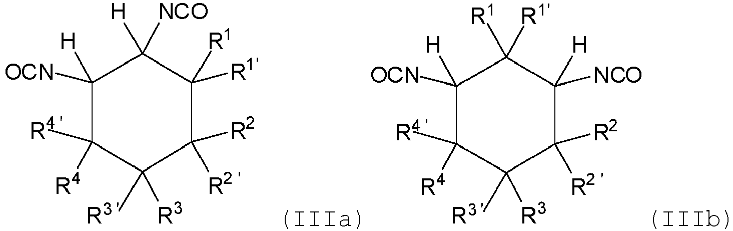 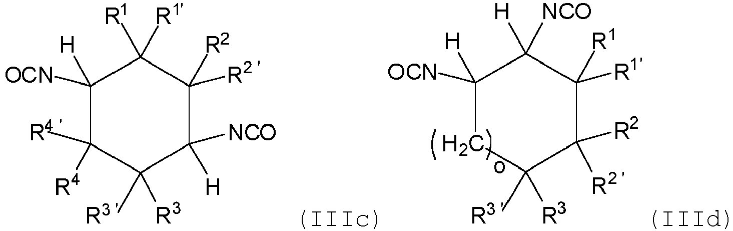 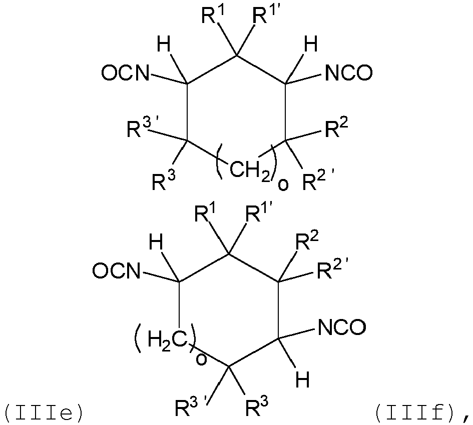 in which
o is 0 to 10, and
R¹, R^{1'}, R², R^{2'}, R³, R^{3'}, R⁴, R^{4'} are each independently selected from the group consisting of H, OR⁵ and unsubstituted or at least monosubstituted C₁-C₁₀-alkyl, C₅-C₁₂-cycloalkyl, C₂-C₁₀-alkenyl and C₆-C₁₄-aryl, where R⁵ is C₁-C₁₀-alkyl, and
(b) at least one alcohol having at least two hydroxyl groups,
to obtain the polyisocyanate (P).

8. The method according to claim 7, wherein component (a) is selected from 1,3-diisocyanatocyclohexane, 1,3-diisocyanato-2-methylcyclohexane, 1,3-diisocyanato-4-methylcyclohexane, 1,3-diisocyanato-5-methylcyclohexane, 1,3-diisocyanato-2-isopropylcyclohexane, 1,3-diisocyanato-4-isopropylcylohexane, 3-diisocyanato-5-isopropylcyclohexane, 1,3-diisocyanato-2,4-dimethylcyclohexane, 1,3-diisocyanato-2,4-diethylcyclohexane, 1,3-diisocyanato-2,4-diethyl-6-methylcyclohexane, 1,3-diisocyanato-2-methyl-4,5-diethylcyclohexane, 1,3-diisocyanato-2,4,6-triisopropylcyclohexane or 1,3-diisocyanato-2,4,6-tributylcyclohexane.

9. The method according to claim 7 or 8, wherein component (b) is selected from ethylene glycol, 1,1-dimethylethylene glycol, 1,2-propanediol, 1,3-propanediol, 2-methyl-1,3-propanediol, 2-ethyl-1,3-propanediol, 2-butyl-2-ethyl-1,3-propanediol, 1,4-butanediol, 2-ethyl-1,4-butanediol, 1,5-pentanediol, 2-methyl-1,5-pentanediol, neopentyl glycol, 1,6-hexanediol, 2-ethyl-1,3-hexanediol, 2,4-diethyloctane-1,3-diol, diethylene glycol, triethylene glycol, tetraethylene glycol, pentaethylene glycol, dipropylene glycol, tripropylene glycol, trimethylolethane, trimethylolpropane, trimethylolbutane, ditrimethylolpropane, pentaerythritol, dipentaerythritol, glycerol, cyclohexane-1,2-diol, cyclohexane-1,3-diol, cyclohexane-1,4-diol, 1,1-bis(hydroxymethyl)cyclohexane, 1,2-bis(hydroxymethyl)cyclohexane, 1,3-bis(hydroxymethyl)cyclohexane, 1,4-bis(hydroxymethyl)cyclohexane, 2,2-bis(4-hydroxycyclohexyl)propane, erythritol, threitol, xylitol, adonitol (ribitol), arabitol (lyxitol), sorbitol, mannitol, dulcitol (galactitol), maltitol, isomalt, diglycerol, dimethylolpropane, dipentaerythritol, ribose, arabinose, glucose, mannose, galactose, fructose, pyrocatechol, catechol, hydroquinone, pyrogallol, hydroxyhydroquinone, phloroglucinol, neopentyl glycol hydroxypivalate, triethanolamine, tripropanolamine, 1,3,5-tris(2-hydroxyethyl)cyanuric acid, polytetrahydrofuran having a molecular weight between 162 and 4500 g/mol, preferably 250 to 2000 g/mol, poly-1,3-propanediol or polypropylene glycol having a molecular weight between 134 and 2000 g/mol or polyethylene glycol having a molecular weight between 238 and 2000 g/mol.

10. The method according to any of claims 7 to 9, wherein component (b) comprises at least two alcohols, wherein at least one alcohol comprises two hydroxyl groups and at least one alcohol comprises at least three hydroxyl groups.

11. The method according to any of claims 7 to 10, wherein the reaction mixture (RM) comprises 70 to 99% by weight component (a) and 1 to 30% by weight component (b), particularly preferably 80 to 95% by weight component (a) and 5 to 20% by weight component (b) and especially preferably 85 to 92% by weight component (a) and 8 to 15% by weight component (b), based on the total weight of components (a) and (b) in the reaction mixture (RM) .

12. The method according to any of claims 7 to 11, wherein
i) the reaction mixture (RM) is reacted at a temperature in the range from 20°C to 90°C, particularly preferably in the range from 40°C to 85°C and especially preferably in the range from 50°C to 80°C, and/or
ii) the reaction mixture (RM) is reacted in the absence of a catalyst.

13. The method according to any of claims 7 to 12, wherein the reaction of the reaction mixture (RM) is terminated by addition of at least one component (c), after at least a portion of components (a) and (b) has been reacted,
wherein the at least one component (c) is preferably selected from toluenesulfonic acid, toluolsulfonyl chloride, benzoyl chloride, benzyl chloride, dibutyl phosphite, dibutyl phosphate or di-(2-ethylhexyl) phosphate.

14. The method according to any of claims 7 to 13, wherein
i) the reaction is terminated after at least one of components (a) or (b), preferably component (b), has been fully reacted, and/or
ii) still unreacted amounts of component (a) and/or (b) are separated off from the composition (C).

15. The use of the polyisocyanate (P) according to any of claims 1 to 6 as crosslinking reagent in clearcoats.

## Revendications

1. Polyisocyanate (P) de formule générale (I) : 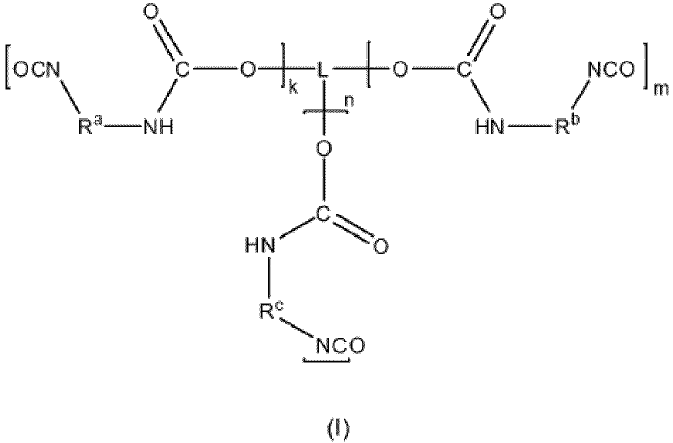 dans laquelle
k, m, n sont indépendamment les uns des autres 0, 1, 2 ou 3, la somme de k, m et n donnant au moins 2 et au plus 6 ; et
L est un radical organique linéaire ou ramifié comportant au plus 14 atomes de carbone, qui peut éventuellement contenir au moins un atome d'azote et/ou au moins un atome d'oxygène,
R^{a}, R^{b}, R^{c} sont choisis indépendamment les uns des autres parmi des radicaux de formules générales (IIa), (IIb), (IIc), (IId), (IIe) et/ou (IIf) : 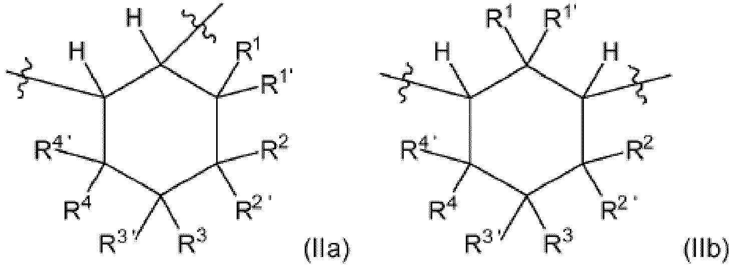 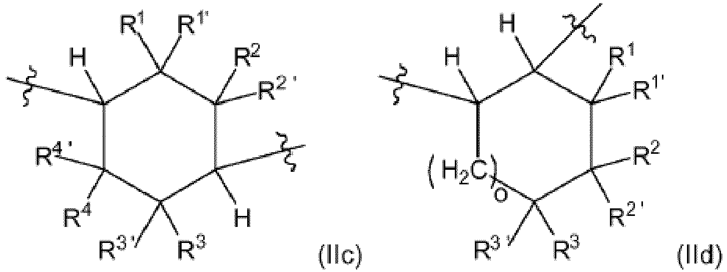 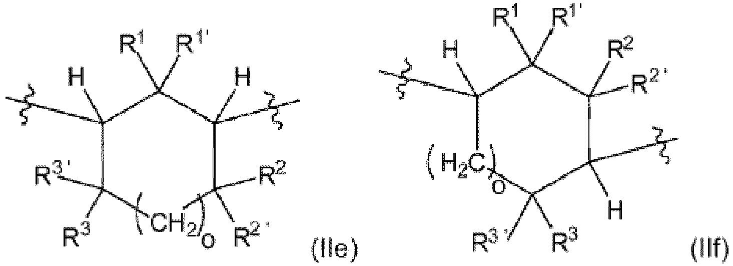 dans lesquelles
o est 0 à 10, et
R¹, R^{1'}, R², R^{2'}, R³, R^{3'}, R⁴, R^{4'} sont choisis indépendamment les uns des autres dans le groupe constitué par H, OR⁵ et C₁-C₁₀-alkyle, C₅-C₁₂-cycloalkyle, C₂-C₁₀-alcényle et C₆-C₁₄aryle non substitué ou au moins monosubstitué, R⁵ étant C₁-C₁₀-alkyle.

2. Polyisocyanate (P) selon la revendication 1, **caractérisé en ce que**
k, m, n sont indépendamment les uns des autres 0, 1, 2 ou 3, la somme de k, m et n donnant au moins 2 et au plus 6 ; et
L est un radical aliphatique linéaire ou ramifié, cycloaliphatique ou aromatique comportant au plus 14 atomes de carbone, qui peut éventuellement contenir au moins un atome d'azote et/ou au moins un atome d'oxygène,
R^{a}, R^{b}, R^{c} sont choisis indépendamment les uns des autres parmi des radicaux de formules générales (IIa), (IIb), (IIc), (IId), (IIe) et/ou (IIf) : 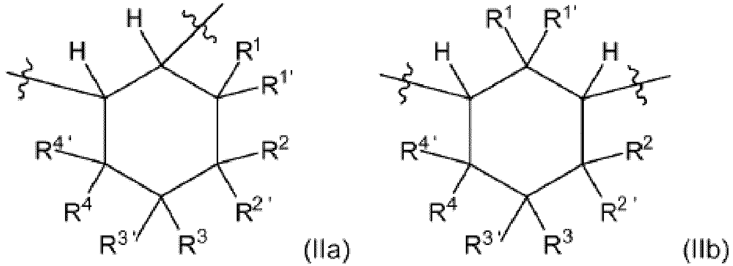 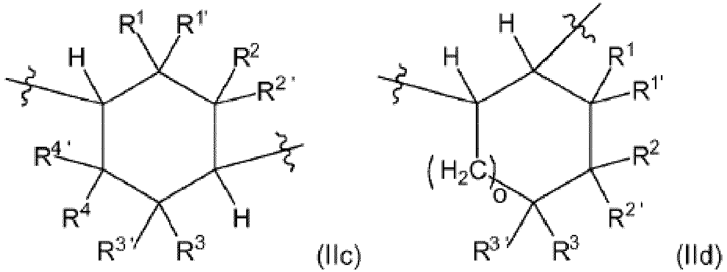 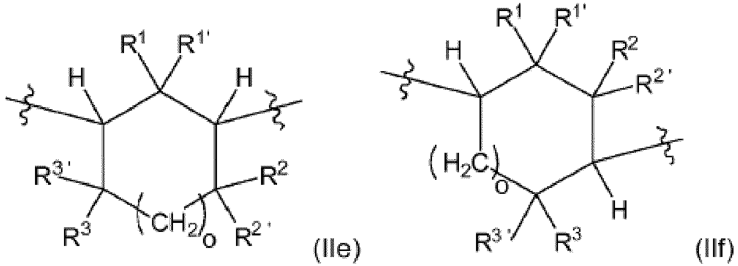 dans lesquelles
o est 0, 2 ou 3 et
R¹, R^{1'}, R², R^{2'}, R³, R^{3'}, R⁴, R^{4'} sont choisis indépendamment les uns des autres dans le groupe constitué par H et C₁-C₁₀-alkyle.

3. Polyisocyanate (P) selon la revendication 1 ou 2, **caractérisé en ce que**
k, m, n sont indépendamment les uns des autres 0, 1, 2 ou 3, la somme de k, m et n donnant au moins 2 et au plus 6 ; et
L est un radical aliphatique linéaire ou ramifié ou cycloaliphatique comportant au plus 14 atomes de carbone, qui peut éventuellement contenir au moins un atome d'azote et/ou au moins un atome d'oxygène,
R^{a}, R^{b}, R^{c} sont choisis indépendamment les uns des autres parmi des radicaux de formules générales (IIa), (IIb) et/ou (IIc) : 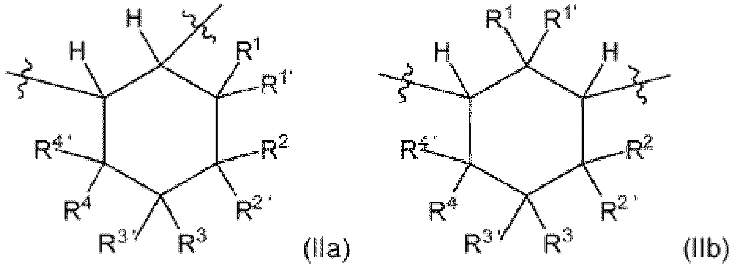 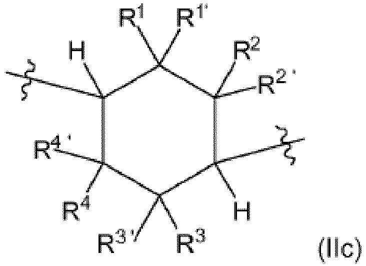 dans lesquelles
R¹, R^{1'}, R², R^{2'}, R³, R^{3'}, R⁴, R^{4'} sont choisis indépendamment les uns des autres dans le groupe constitué par H, méthyle, éthyle et propyle.

4. Polyisocyanate (P) selon l'une quelconque des revendications 1 à 3, **caractérisé en ce que**
k est 2 ou 3, et
m, n sont 0, et
L est un radical aliphatique linéaire ou ramifié comportant au plus 10 atomes de carbone, qui peut éventuellement contenir au moins un atome d'azote et/ou au moins un atome d'oxygène,
R^{a} est choisi parmi des radicaux de formules générales (IIa), (IIb) et/ou (IIc) : 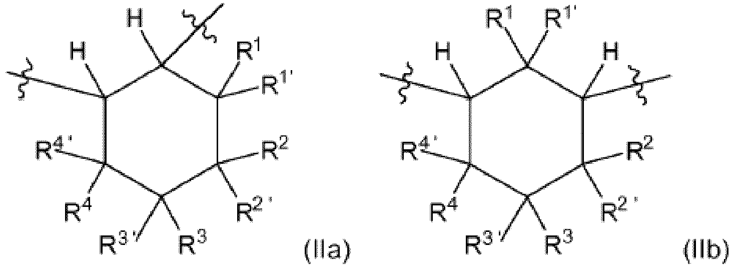 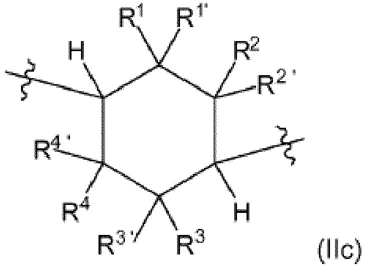 dans lesquelles
R¹, R^{1'}, R², R^{2'}, R³, R^{3'}, R⁴, R^{4'} sont choisis indépendamment les uns des autres dans le groupe constitué par H, méthyle et éthyle,
au moins un radical R¹, R^{1'}, R², R^{2'}, R³, R^{3'}, R⁴, ou R^{4'} étant méthyle ou éthyle, de préférence méthyle.

5. Polyisocyanate (P) selon l'une quelconque des revendications 1 à 4, **caractérisé en ce que**
k est 3, et
m, n sont 0, et
L est un radical aliphatique linéaire ou ramifié comportant au plus 6 atomes de carbone, qui peut éventuellement contenir au moins un atome d'oxygène,
R^{a} est un radical de formule générale (IIb) : 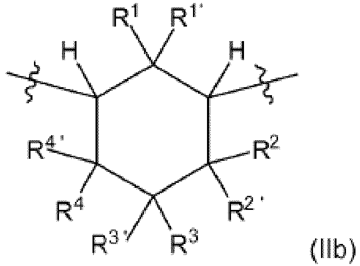 dans laquelle
R¹, R^{1'}, R², R^{2'}, R³, R^{3'}, R⁴, R^{4'} sont choisis indépendamment les uns des autres dans le groupe constitué par H, méthyle et éthyle, au moins un radical et au plus 3 radicaux R¹, R^{1'}, R², R^{2'}, R³, R^{3'}, R⁴ ou R^{4'} étant méthyle ou éthyle, de préférence méthyle.

6. Polyisocyanate (P) de formule générale (IV) 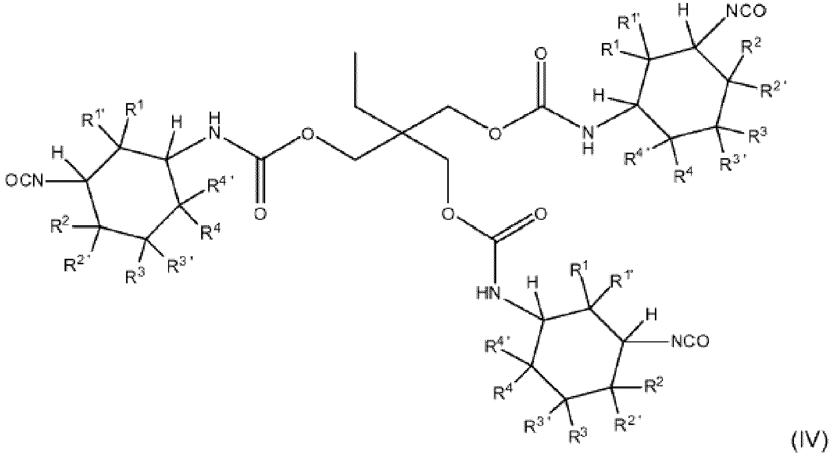 dans laquelle
R¹, R^{1'}, R², R^{2'} sont choisis indépendamment les uns des autres dans le groupe constitué par H et méthyle, au moins un radical et au plus deux radicaux R¹, R^{1'}, R² ou R^{2'} étant méthyle, et
R³, R^{3'}, R⁴, R^{4'} sont H.

7. Procédé pour la préparation du polyisocyanate (P) selon l'une quelconque des revendications 1 à 6, comprenant la transformation d'un mélange réactionnel (MR) contenant les composants suivants (a) et (b) :
(a) au moins un isocyanate cyclique de formules générales (IIIa), (IIIb), (IIIc), (IIId), (IIIe) et/ou (IIIf) : 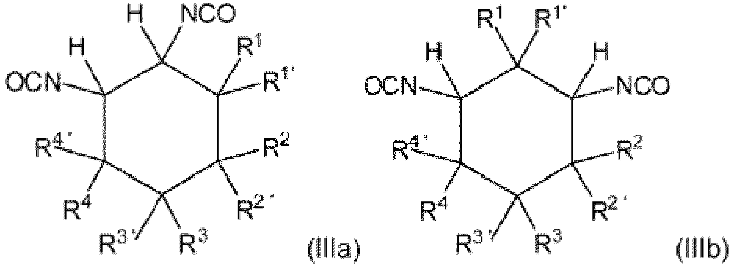 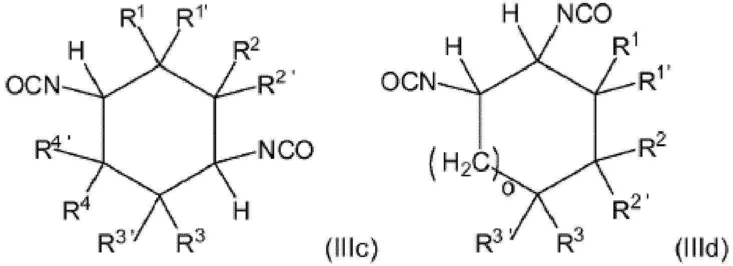 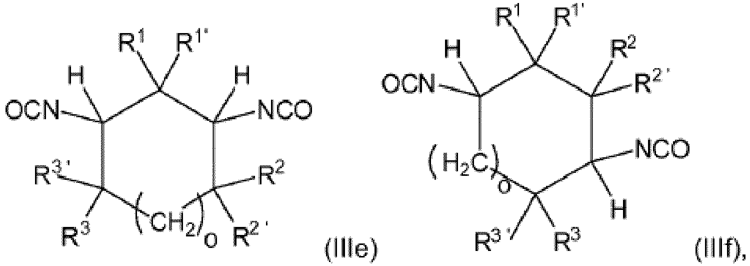 dans lesquelles
o est 0 à 10, et
R¹, R^{1'}, R², R^{2'}, R³, R^{3'}, R⁴, R^{4'} sont choisis indépendamment les uns des autres dans le groupe constitué par H, OR⁵ et C₁-C₁₀-alkyle, C₅-C₁₂-cycloalkyle, C₂-C₁₀-alcényle et C₆-C₁₄-aryle non substitué ou au moins monosubstitué, R⁵ étant C₁-C₁₀-alkyle, et
(b) au moins un alcool, qui présente au moins deux groupes hydroxy,
pour obtenir le polyisocyanate (P).

8. Procédé selon la revendication 7, **caractérisé en ce que** le composant (a) est choisi parmi le 1,3-diisocyanatocyclohexane, le 1,3-diisocyanato-2-méthylcyclohexane, le 1,3-diisocyanato-4-méthylcyclohexane, le 1,3-diisocyanato-5-méthylcyclohexane, le 1,3-diisocyanato-2-isopropylcyclohexane, le 1,3-diisocyanato-4-isopropylcylohexane, le 3-diisocyanato-5-isopropylcyclohexane, le 1,3-diisocyanato-2,4-diméthylcyclohexane, le 1,3-diisocyanato-2,4-diéthylcyclohexane, le 1,3-diisocyanato-2,4-diéthyl-6-méthylcyclohexane, le 1,3-diisocyanato-2-méthyl-4,5-diéthylcyclohexane, le 1,3-diisocyanato-2,4,6-triisopropylcyclohexane et le 1,3-diisocyanato-2,4,6-tributylcyclohexane.

9. Procédé selon la revendication 7 ou 8, **caractérisé en ce que** le composant (b) est choisi parmi l'éthylèneglycol, le 1,1-diméthyléthylèneglycol, le 1,2-propanediol, le 1,3-propanediol, le 2-méthyl-1,3-propanediol, le 2-éthyl-1,3-propanediol, le 2-butyl-2-éthyl-1,3-propanediol, le 1,4-butanediol, le 2-éthyl-1,4-butanediol, le 1,5-pentanediol, le 2-méthyl-1,5-pentanediol, le néopentylglycol, le 1,6-hexanediol, le 2-éthyl-1,3-hexanediol, le 2,4-diéthyloctane-1,3-diol, le diéthylèneglycol, le triéthylèneglycol, le tétraéthylèneglycol, le pentaéthylèneglycol, le dipropylèneglycol, le tripropylèneglycol, le triméthyloléthane, le triméthylolpropane, le triméthylolbutane, le ditriméthylolpropane, le pentaérythritol, le dipentaérythritol, la glycérine, un cyclohexane-1,2-diol, un cyclohexane-1,3-diol, un cyclohexane-1,4-diol, le 1,1-bis(hydroxyméthyl)cyclohexane, le 1,2-bis(hydroxyméthyl)cyclohexane, le 1,3-bis(hydroxyméthyl)cyclohexane, le 1,4-bis(hydroxyméthyl)cyclohexane, le 2,2-bis(4-hydroxycyclohexyl)propane, l'érythritol, le thréitol, le xylitol, l'adonitol (ribitol), l'arabitol (lyxitol), le sorbitol, le mannitol, le dulcitol (galactitol), le maltitol, l'isomalt, la diglycérine, le diméthylolpropane, le dipentaérythritol, le ribose, l'arabinose, le glucose, le mannose, le galactose, le fructose, la brenzcatéchine, le catéchol, l'hydroquinone, le pyrogallol, l'hydroxyhydroquinone, la phloroglucine, l'ester de néopentylglycol de l'acide hydroxypivalique, la triéthanolamine, la tripropanolamine, l'acide 1,3,5-tris(2-hydroxyéthyl)cyanurique, un polytétrahydrofuranne doté d'un poids moléculaire compris entre 162 et 4 500 g/mole, préférablement de 250 à 2 000 g/mole, un poly-1,3-propanediol ou un polypropylèneglycol doté d'un poids moléculaire compris entre 134 et 2 000 g/mole ou un polyéthylèneglycol doté d'un poids moléculaire compris entre 238 et 2 000 g/mole.

10. Procédé selon l'une quelconque des revendications 7 à 9, **caractérisé en ce que** le composant (b) comprend au moins deux alcools, au moins un alcool présentant deux groupes hydroxy et au moins un alcool présentant au moins trois groupes hydroxy.

11. Procédé selon l'une quelconque des revendications 7 à 10, **caractérisé en ce que** le mélange réactionnel (MR) contient 70 à 99 % en poids du composant (a) et 1 à 30 % en poids du composant (b), particulièrement préférablement 80 à 95 % en poids du composant (a) et 5 à 20 % en poids du composant (b) et tout particulièrement préférablement 85 à 92 % en poids du composant (a) et 8 à 15 % en poids du composant (b), par rapport au poids total des composants (a) et (b) dans le mélange réactionnel (MR).

12. Procédé selon l'une quelconque des revendications 7 à 11, **caractérisé en ce que**
i) la transformation du mélange réactionnel (MR) est réalisée à une température dans la plage de 20 à 90 °C, particulièrement préférablement dans la plage de 40 à 85 °C et tout particulièrement préférablement dans la plage de 50 à 80 °C, et/ou
ii) la transformation du mélange réactionnel (MR) est réalisée en l'absence d'un catalyseur.

13. Procédé selon l'une quelconque des revendications 7 à 12, **caractérisé en ce que** la transformation du mélange réactionnel (MR) est stoppée par l'ajout d'au moins un composant (c), après qu'au moins une partie des composants (a) et (b) a été transformée,
l'au moins un composant (c) étant de préférence choisi parmi l'acide toluènesulfonique, le chlorure d'acide toluènesulfonique, le chlorure de benzoyle, le chlorure de benzyle, le phosphite de dibutyle, le phosphate de dibutyle et le phosphate de di-(2-éthylhexyle).

14. Procédé selon l'une quelconque des revendications 7 à 13, **caractérisé en ce que**
i) la transformation est stoppée après qu'au moins l'un des composants (a) et (b), de préférence le composant (b), a été complètement transformé, et/ou
ii) des quantités du composant (a) et/ou (b) n'ayant pas encore réagi sont séparées de la composition (Z).

15. Utilisation du polyisocyanate (P) selon l'une quelconque des revendications 1 à 6 en tant que réactif de réticulation dans des vernis transparents.
